# EUROPEAN PATENT SPECIFICATION

(11) **EP 3 380 080 B1**
(45) Date of publication and mention of the grant of the patent: **22.09.2021**
(21) Application number: 16869283.8
(22) Date of filing: 23.11.2016
(51) Int. Cl.: A61K 31/167, A61K 31/44, A61K 47/10, A61K 47/32, A61K 47/40, A61K 47/44, A61K 31/7048, A61K 31/7036, A61K 31/566, A61K 31/4468, A61K 31/4458, A61K 31/445, A61K 31/135, A61K 31/568, A61K 47/38, A61K 47/26, A61K 47/08, A61K 47/12, A61K 47/14, A61K 45/06, A61P 23/02

(54) **TOPICAL FILM-FORMING SPRAY**
TOPISCHES FILMBILDENDES SPRAY
PULVÉRISATION FILMOGÈNE TOPIQUE

(30) Priority: 23.11.2015 US 201562258672 P
(43) Date of publication of application: 03.10.2018
(73) Proprietor: Nortic Holdings Inc., East Brunswick, NJ 08816 (US)
(72) Inventor: KOTTAYIL, S., George, West Windsor, NJ 08550 (US); KUMAR, Amresh, Plainsboro, NJ 08536 (US); SUNTHANKAR, Prasanna, West Windsor, NJ 08550 (US); KAVURU, Vimal, Holmdel, NJ 07733 (US)
(74) Representative: Dehns
(86) International application number: PCT/US2016/063618
(87) International publication number: WO 2017/091739

(56) References cited:
- WO-A1-2007/002518
- WO-A1-2014/130846
- WO-A2-00/45795
- CN-A- 104 013 574
- US-A1- 2007 219 171
- US-A1- 2009 060 986
- US-A1- 2009 191 271
- US-A1- 2012 177 697
- US-A1- 2012 214 776
- US-A1- 2013 195 965
- US-A1- 2014 212 362
- US-A1- 2015 157 729
- US-A1- 2015 157 729
- LU , W et al.: "Preparation and characterization of a metered dose transdermal spray for testosterone", Acta Pharmaceutica Sinica B, vol. 3, no. 6, 2013, pages 392-399, XP055596081,
- SIVAKUMARAN, DN: Synthesis of degradable thermoresponsive microgels, September 2015 (2015-09), XP055596083, Hamilton, Ontario University Retrieved from the Internet: URL:https://macsphere.mcmaster.ca/bitstrea m/11375/18303/2/Sivakumaran_Daryl_N_2015Se pt_PhD.pdf [retrieved on 2017-11-01]

## Description

### FIELD OF THE INVENTION

The present invention relates to a film-forming composition which forms a stable film when applied (e.g., sprayed) on a surface (e.g., skin). The stable film is formed by evaporation of solvent and adheres to the surface.

### BACKGROUND OF THE INVENTION

Topical films that incorporate therapeutic agents have been developed for a variety of purposes in the pharmaceutical arts. For example, Tipton et al. (U.S. Pat. Nos. 5,632,727 and 5,792,469) describes the preparation of a biodegradable film dressing with or without additional therapeutic agents formed from a liquid composition of at least one biodegradable/bioerodible thermoplastic polymer in a pharmaceutically acceptable solvent. The film is formed by dispensing, preferably by spraying, the liquid composition onto a tissue site and contacting the liquid composition with an aqueous-based fluid to coagulate or solidify the film onto the human or animal tissue. The biodegradable film can be used to protect and to promote healing of injured tissue and/or to deliver biologically active agents.

U.S. Pat. No. 6,958,154 describes a spray-on bandage and drug delivery system. Therein, a fluid composition, e.g., an aerosol spray, is applied onto a surface as a fluid, but then dries in situ to form a patch having a tack-free outer surface covering an underlying adhesive that helps to adhere the patch to the substrate.

U.S. Pat. No. 6,899,897 describes the use of a natural gum resin as a carrier for topical application of an active agent. The biological dressing described therein is comprised of a gum resin, a topically acceptable volatile solvent, and a pharmacologically active agent. The gum resin is present in a suitable amount that the composition, when the solvent evaporates, will dry to form a solid coating that sticks to the skin or mucosal membrane to which the composition is applied and maintain the pharmacologically active agent over a sustained period of time in contact with sites on the skin or mucosal membranes exhibiting symptoms of a disease to be treated.

US 2007/0219171 describes transdermal pharmaceutical spray formulations which contain a pharmaceutically active agent, a VP/VA copolymer, and a non-aqueous vehicle. The formulations further comprise an anti-nucleating agent to prevent recrystallization of the pharmaceutically active agent and a penetration enhancer to increase the rate of drug delivery through the skin. Upon application to the skin, the formulations dry to provide a film at the site of treatment.

However, conventional topical spray formulations tend to remain at the application site for only a short time and as a result, the active agent is absorbed through the skin is only available transiently. Further, known topical spray formulations may cause skin irritation or occlusion problems.

### OBJECTS AND SUMMARY OF THE INVENTION

It is an object of the present disclosure to provide formulations and methods for providing pain relief in humans and animals by administering a dose of a local anesthetic formulation to an open wound or surgical site for the treatment of acute or chronic pain, nociceptive and neuropathic pain.

It is an object of the present disclosure to provide formulations and methods for providing pain relief in humans and animals by administering a dose of a local anesthetic formulation for the treatment of pre- and post-operative pain, cancer pain, pain associated with neurotransmitter dysregulation syndromes and orthopedic disorders, and/or localized severe or intractable pain.

The present disclosure in certain aspects is directed to a method of treating pain comprising the administration of a polymeric film forming spray formulation which is applied to the skin via the utilization of a (mechanical pump) spray device.

The invention is directed to a polymeric film forming topical spray formulation, comprising a hydrophilic film forming polymer, an active agent, a drug crystal precipitation inhibiting agent, wherein the hydrophilic film forming polymer and the drug crystal precipitation inhibiting agent are the same and it is povidone in an amount from 2.5 to 20% by weight, the amount of povidone being effective to prevent or substantially prevent the active agent included in the formulation from precipitating, a pharmaceutically acceptable permeation enhancer for the active agent, and a volatile solvent in a concentration from 20 to 99% of the formulation, by weight, the formulation when sprayed on and set on a site on human skin providing a breathable, bioadhesive and microporous film in which the active agent is supersaturated and further providing a biphasic release of the active agent(s) such that the formulation provides a first peak concentration of the active agent at 0.25 to 1.5 hours after application of the topical spray on the skin of a human subject, and provides a second peak concentration of the active agent at 4 hours to 12 hours after application of the topical spray on the skin of a human subject. In certain preferred embodiments, the topical spray formulation provides an initial release of active agent(s) once the topical spray is sprayed onto human skin followed by a lag during which less drug is released over a period of time. In certain preferred embodiments, the formulation provides a cumulative drug permeation in-vitro from 10 µg/cm² to 6500 µg/cm², performed on Mattek Epiderm or cadaver skin using In-line PermeGear ILC07 automatic diffusion cell system. The formulation comprises a supersaturated concentration of the drug (e.g., bupivacaine). This may result in the drug concentration lasting for an extended period of time in vivo (e.g., from 1 to 24 hours, or from 3 to 12 hours) and may provide increased bioavailability of the drug from the formulation. In certain preferred embodiments, the topical polymeric film forming composition provides an in-vitro cumulative drug permeation from 10 µg/cm² to 500 µg/cm² after 2 hours, and a cumulative in-vitro drug permeation from 10 µg/cm² to 6500 µg/cm² after 24 hours. In other preferred embodiments, the topical polymeric provides an in-vivo cumulative drug permeation on human skin from 10 ng/cm² to 500 ng/cm² after 2 hours, and a cumulative drug permeation in-vivo from 10 ng/cm² to 6500 ng/cm² after 24 hours. The topical spray formulation provides a biphasic release of the active agent(s) in which a first portion of the active agent(s) is released either immediately or after a short time delay to provide a first peak maximum concentration (i) at the site when the drug is topically active, or (ii) in blood plasma when the drug is systemically active which occurs from 0.25 hours to 1.5 hours after application of the topical spray on the skin, and a second portion of the active agent(s) released after a lag time to provide a second peak maximum concentration from 4 hours to 12 hours after application of the topical spray on the skin.

In certain embodiments, the formulation provides a cumulative in-vitro drug (e.g., bupivacaine) permeation from 10 µg/cm² to 6500 µg/cm², performed for example on Mattek Epiderm or cadaver skin using In-line PermeGear ILC07 automatic diffusion cell system.

The present invention is further directed in part to a unit dose of a topical spray pharmaceutical formulation comprising a polymeric solution, emulsion or suspension of povidone in an amount from 2.5 to 20% by weight, a therapeutically effective amount of a supersaturated active agent, and a pharmaceutically acceptable permeation enhancer dispersed in a pharmaceutically acceptable hydroalcoholic solvent, the unit dose comprising a plurality of spray droplets, wherein 10% of the spray droplets in the unit dose have a mean diameter of 26 µm ± 20 µm, 50% of the spray droplets in the unit dose have a mean diameter of 55 µm ± 20 µm, and 90% of the spray droplets in the unit dose have a mean diameter of 116 µm ± 40 µm, the unit dose topical spray provides a film surface area from 1 cm² to 40 cm² per spray and sets as a microporous, breathable and bioadhesive film when the hydroalcoholic solvent evaporates and provides a biphasic release of the supersaturated active agent.

The present invention in certain embodiments is directed to a topical polymeric film forming composition, comprising bupivacaine hydrochloride dispersed in a hydroalcoholic solvent together with a hydrophilic film forming polymer, a drug crystal precipitation inhibiting agent in an amount effective to prevent or substantially prevent the drug(s) included in the formulation from precipitating, and a pharmaceutically acceptable permeation enhancer for the active agent(s) and wherein the hydrophilic film forming polymer and the drug crystal precipitation inhibiting agent are the same and it is povidone in an amount of from 2.5 to 20% by weight, and a permeation enhancer, the formulation being capable of being sprayed as a unit dose onto skin via the use of a pump spray to provide droplets having a diameter from about 1 to about 1000 microns, when sprayed and set on skin providing a breathable, bioadhesive and microporous film. In certain preferred embodiments, the topical polymeric film forming composition, which when sprayed and set on human skin (e.g., to an affected area of the human) releases from about 0.001% to about 25% of the bupivacaine within about 2 hours, and further providing a biphasic release of the bupivacaine. In certain preferred embodiments, the topical polymeric film forming composition provides an in-vitro cumulative drug permeation from 10 µg/cm² to 500 µg/cm² after 2 hours, and a cumulative in-vitro drug permeation from 10 µg/cm² to 6500 µg/cm² after 24 hours. In other preferred embodiments, the topical polymeric provides an in-vivo cumulative drug permeation on human skin from 10 ng/cm² to 500 ng/cm² after 2 hours, and a cumulative drug permeation in-vivo from 10 ng/cm² to 6500 ng/cm² after 24 hours. The topical polymeric film forming composition provides a biphasic release profile in-vivo. The topical spray formulation provides a biphasic release of the active agent(s) in which a first portion of the active agent(s) is released either immediately or after a short time delay to provide a first peak maximum concentration (i) at the site when the drug is topically active), or (ii) in blood plasma when the drug is systemically active which occurs from 0.25 hours to 1.5 hours after application of the topical spray on the skin, and a second portion of the active agent(s) released after a lag time to provide a second peak maximum concentration from 4 hours to 12 hours after application of the topical spray on the skin. In preferred embodiments, the topical polymeric film forming composition provides a biphasic release when sprayed and set on human skin and having a first and second phase of release, wherein the first phase of drug release reaches a peak at from 0.5 to 1.5 hours and the second phase of release reaches a peak plasma concentration at 3 to 7 hours after application of the unit dose on human skin. In certain embodiments, the formulation provides a bupivacaine dose from about 0.5 to about 40 mg, or from about 1 mg to about 20 mg bupivacaine, sprayed as a unit dose onto the skin, to provide a film surface area from about 1 cm² to about 40 cm² per spray. The unit dose may be comprised, e.g., of from 1 to about 20 sprays per application of the unit dose onto the skin.

The invention is also directed to a unit dose of a topical spray pharmaceutical formulation comprising a polymeric solution, emulsion or suspension of povidone in an amount from 2.5 to 20% by weight, a therapeutically effective amount of a supersaturated active agent, and a pharmaceutically acceptable permeation enhancer dispersed in a pharmaceutically acceptable hydroalcoholic solvent, the unit dose comprising a plurality of spray droplets, wherein 10% of the spray droplets in the unit dose have a mean diameter of 26 µm ± 20 µm, 50% of the spray droplets in the unit dose have a mean diameter of 55 µm ± 20 µm, and 90% of the spray droplets in the unit dose have a mean diameter of 116 µm ± 40 µm, the unit dose topical spray provides a film surface area from 1 cm² to 40 cm² per spray and sets as a microporous, breathable and bioadhesive film when the hydroalcoholic solvent evaporates and provides a biphasic release of the supersaturated active agent. In certain embodiments, a dose of the active agent is sprayed as a unit dose onto the skin, to provide a film surface area, e.g., from about 1 cm² to about 40 cm², and in certain embodiments preferably about 20 cm² per spray. The unit dose may be administered by spraying from about 1 to about 20 sprays (which would be, e.g., 20 x 20 cm²) or from 1 to about 7 sprays per application of the unit dose onto the skin. In certain preferred embodiments, the biphasic release provides a first peak concentration of the active agent at from 0.5 to 3 hours and the second peak concentration of the active agent at from 3 to 15 hours after application of the unit dose on human skin. In certain embodiments, the biphasic release provides a first peak at from 0.5 to 3 hours and a second peak at from 3 to 7 hours after application of the unit dose on human skin. In certain preferred embodiments, 10% of the spray droplets in the unit dose have a mean diameter of about 26 µm ± 1.82 µm, about 50% of the spray droplets in the unit dose have a mean diameter of about 55 µm ± 2.39 µm, and about 90% of the spray droplets in the unit dose have a mean diameter of about 116 µm ± 4.9 µm.

In certain embodiments, the active agent comprises from 0.5 to 40 mg bupivacaine hydrochloride. The bupivacaine concentration in the formulation is supersaturated. The supersaturated drug concentration may last for a time period from about 1 to about 24 hours in vivo, to achieve increased bioavailability. In certain preferred embodiments, the first peak concentration of bupivacaine occurs at from 0.17 to 0.67 hours after the unit dose is sprayed onto the human subject, and the second peak concentration of bupivacaine occurs at from 4 to 24 hours after the unit dose is sprayed onto the human subject. In certain embodiments, the unit dose provides a first peak plasma concentration from 29 pg/ml to 380 pg/ml bupivacaine, and a second peak plasma concentration from 864 pg/ml to 3463 pg/ml bupivacaine. The unit dose containing the bupivacaine may be, e.g., 12 mg, and the dose may be administered as 12 mg per spray.

In certain aspects, the disclosure is directed to a method of treating neuropathic pain, comprising spraying a unit dose of a topical polymeric film forming composition comprising a therapeutically effective amount of a local anesthetic (e.g., bupivacaine hydrochloride) dispersed in a hydroalcoholic solvent together with a hydrophilic film forming polymer in an amount from about 2 to about 50%, by weight, an effective amount of drug crystal precipitation inhibiting agent, and a permeation enhancer onto the skin of a human patient, such that the unit dose is sprayed onto the skin in the form of droplets having a diameter from about 1 to about 1000 microns and the droplets, when set on the skin providing a breathable, bioadhesive and microporous film which provides a biphasic release of the bupivacaine. In certain aspects, a bupivacaine dose from about 1 to about 20 mg is sprayed as a unit dose onto the skin, to provide a film surface area from about 1 cm² to about 40 cm², preferably about 20 cm² per spray, further comprising spraying from about 1 to about 20 sprays or from 1 to about 7 sprays per application of the unit dose onto the skin. In certain aspects, the method further comprises spraying a further unit dose of the topical polymeric film forming composition onto the skin of the human patient about 6 hours after application of a first unit dose. In certain aspects, the unit dose is sprayed using a metered pump delivering from about 40 µl to about 350 µl volume per spray. In certain aspects, the biphasic release provides a first and second phase of release, wherein the first phase of bupivacaine release reaches a peak at about 0.5 to about 3 hours and the second phase of bupivacaine release reaches a peak plasma concentration at about 3 to about 15 hours or at about 3 to about 7 hours after application of the unit dose on human skin.

In certain aspects, the bupivacaine topical spray droplet size from about 20 to about 31 µm Dv (10), and/or a spray droplet size from about 49 to about 60 µm Dv (50), and/or a topical spray droplet size from about 100 to about 140 µm Dv (90).

The disclosure is further directed in part to a method of treating neuropathic pain (e.g., erythromelalgia, post-herpetic neuralgia, fibromyalgia and/or complex regional pain syndrome (CRPS), among others) comprising spraying a unit dose of a topical polymeric film forming composition comprising a local anesthetic (e.g., bupivacaine base, bupivacaine hydrochloride or a mixture thereof) dispersed in a hydroalcoholic solvent together with a hydrophilic film forming polymer in an amount from about 2 to about 50%, by weight, an effective amount of a drug crystal precipitation inhibiting agent, and a permeation enhancer onto the skin of a human patient, such that the unit dose containing a therapeutically effective amount of the local anesthetic is sprayed onto the skin in the form of droplets having a diameter from about 1 to about 1000 microns and the droplets, when set on the skin providing a breathable, bioadhesive and microporous film which provides a biphasic release of the bupivacaine. The bupivacaine dose may be from about 0.5 to about 40 mg is sprayed as a unit dose onto the skin, to provide a film surface area from about 1 cm² to about 40 cm² per spray, and may be dosed by spraying from 1 to about 20 sprays per application of the unit dose onto the skin. In certain aspects, a further unit dose of the topical polymeric film forming composition may be sprayed onto the skin of the human patient about 6 hours after application of a first unit dose. The unit dose may be sprayed using a metered pump delivering, e.g., from about 40 µl to about 350 µl volume per spray. The biphasic release preferably provides a first and second phase of release, wherein the first phase of bupivacaine release reaches a peak at from about 0.25 to about 1.5 hours and the second phase of bupivacaine release reaches a peak plasma concentration at from about 4 to about 12 hours after application of the unit dose on human skin.

The disclosure is also directed in part to a method of treating pain, comprising spraying onto the skin of a human a unit dose of a topical spray pharmaceutical formulation comprising a polymeric solution, emulsion or suspension of a hydrophilic polymer, a drug crystal precipitation inhibiting agent, an effective amount of an active agent suitable for treating pain, and a pharmaceutically acceptable permeation enhancer dispersed in a pharmaceutically acceptable hydroalcoholic solvent, the unit dose comprising a plurality of spray droplets, wherein 10% of the spray droplets in the unit dose have a mean diameter of about 26 µm ± 20 µm, about 50% of the spray droplets in the unit dose have a mean diameter of about 55 µm ± 20 µm, and about 90% of the spray droplets in the unit dose have a mean diameter of about 116 µm ± 40 µm, such that the unit dose topical spray provides a film surface area from about 1cm² to about 40 cm² per spray and sets as a microporous, breathable and bioadhesive film when the hydroalcoholic solvent evaporates and provides a biphasic release of the active agent.

The disclosure is further directed in part to a method of treating pain, comprising spraying onto the skin of a human in proximity to an affected area a unit dose of a topical spray pharmaceutical formulation comprising a polymeric solution, emulsion or suspension of a hydrophilic polymer, a drug crystal precipitation inhibiting agent, an effective amount of an active agent comprising a local anesthetic or a pharmaceutically acceptable salt thereof, and a pharmaceutically acceptable permeation enhancer dispersed in a pharmaceutically acceptable hydroalcoholic solvent, the unit dose comprising a plurality of spray droplets, wherein 10% of the spray droplets in the unit dose have a mean diameter of about 26 µm ± 20 µm, about 50% of the spray droplets in the unit dose have a mean diameter of about 55 µm ± 20 µm, and about 90% of the spray droplets in the unit dose have a mean diameter of about 116 µm ± 40 µm, such that the unit dose topical spray sets as a microporous, breathable and bioadhesive film having a film surface area from about 1cm² to about 40 cm² per spray and when the hydroalcoholic solvent evaporates and provides a biphasic release of the active agent.

In certain aspects, the methods further comprise using a metered pump delivering, e.g., from about 40 µl to about 350 µl volume per spray to deliver the active agent onto the skin of the human.

Any methods of medical treatment herein disclosed do not form part of the invention.

For purposes of the present invention, the terms "active agent", "drug" and medicament are used interchangeably, and are meant to encompass a single drug or multiple drugs (two or more) contained in the topical spray formulations of the invention.

The term "local anesthetic" means any drug or mixture of drugs that provides local numbness and/or analgesia.

As used herein, the term "unit dose" refers to physically discrete units suitable as unitary dosages for mammalian subjects, each unit containing as the active ingredient a predetermined quantity of the active agent (e.g., local anesthetic).

The term "comprising" is an inclusive term interpreted to mean containing, embracing, covering or including the elements listed following the term, but not excluding other unrecited elements.

For purposes of the invention, the term "controlled release", "sustained release", and similar terms are used to denote a mode of active agent delivery that occurs when the active agent is released from the delivery vehicle at an ascertainable and controllable rate over a period of time, rather than dispersed immediately upon application or injection. The controlled or sustained release formulations of the invention preferably provide a sustained action in the localized area to be treated. For example, it would be desirable that such a formulation provides localized anesthesia to the site for a period of, e.g., one day. The formulations can therefore, of course, be modified in order to obtain such a desired result.

The term "modified release" as used herein in relation to the composition according to the invention or a coating or coating material or used in any other context means release which is not immediate release and is taken to encompass controlled release, sustained release and delayed release.

A "therapeutically effective amount" means the amount that, when administered to an animal for treating a disease, is sufficient to effect treatment for that disease.

As used herein, the term "treating" or "treatment" of a disease includes preventing the disease from occurring in an animal that may be predisposed to the disease but does not yet experience or exhibit symptoms of the disease (prophylactic treatment), inhibiting the disease (slowing or arresting its development), providing relief from the symptoms or side-effects of the disease (including palliative treatment), and relieving the disease (causing regression of the disease). For the purposes of this disclosure, a "disease" includes pain.

As used herein, the term "bioerodible" refers to the degradation, disassembly or digestion of the sustained release carrier of the gel formulation by action of a biological environment, including the action of living organisms and most notably at physiological pH and temperature.

As used herein, the term "topically active" refers to a film formulation in accordance with the present invention which includes at least one active agent which treats predominately the surface on which it is applied.

As used herein, the term "transdermal" refers to a film formulation in accordance with the present invention which includes at least one active agent that is absorbed through skin when applied topically. Typically, although not necessarily, the medicament will then be distributed throughout the body resulting in systemic action as opposed to being only locally active at the site of application.

For purposes of the present invention, the term "active agent" is meant to encompass, but not be limited to, a drug. The term active agent is further meant to encompass a single active agent, or multiple (two or more) active agents present in the formulation.

For purposes of the present invention, all percentages described herein are "w/w" unless otherwise specified.

### BRIEF DESCRIPTION OF THE DRAWINGS

Figure 1 depicts an in-vivo drug plasma concentration vs time plot of individual rats treated with a bupivacaine (HCl and base) metered dose transdermal film sprays in accordance with the present invention.
Figure 2 depicts an in-vivo drug plasma concentration vs time plot of individual rats treated with a bupivacaine metered dose transdermal film spray in accordance with the present invention.
Figure 3 depicts an in-vivo average drug plasma concentration vs time plot of 8 mg, 12 mg and placebo of individual rats treated with a bupivacaine metered dose transdermal film spray in accordance with the present invention.
Figure 4 depicts an in-vivo average cumulative drug plasma concentration vs time plot of 8 mg, 12 mg and placebo of individual rats treated with a bupivacaine metered dose transdermal film spray in accordance with the present invention.
Figure 5 depicts bupivacaine topical spray film characteristics of bupivacaine spray formulations containing different concentrations of PVP, as compared to a bupivacaine and ethanol spray.
Figure 6 depicts the spray pattern and film uniformity of bupivacaine spray formulations according to the present invention containing different concentrations of PVP.

### DETAILED DESCRIPTION

The polymeric film forming preparations produce a stable film in situ after application on the skin or any other body surface. These compositions can either be liquids or semisolids with a hydrophilic film forming polymer and optional hydrophobic film forming polymer as basic material for the matrix. The compositions include a hydrophilic film forming polymer which is povidone in an amount from 2 to 20% by weight. The formed film is sufficiently stable to provide a drug release to the skin from 1 hour up to 24 hours. The polymeric solution is sprayed to the skin as a liquid and forms an almost invisible film in situ by volatile solvent evaporation. The film is peal-able and/or non-pealable in nature. The film is microporous. The film is breathable, meaning that it does not interfere with perspiration, respiration and other metabolic activities of the skin. The film is preferably non-greasy and non-sticky. It is also preferably non-transferable. It is bioadhesive.

The present invention is directed in certain embodiments to a film forming topical spray composition containing an active agent (such as bupivacaine or a pharmaceutically acceptable salt thereof). The composition comprises the active agent, a hydrophilic film forming polymer which is povidone, a volatile solvent, and a permeation enhancer (e.g., propylene glycol, oleyl alcohol, polyethylene glycol ethers of oleyl alcohol (e.g., Oleth-2), combinations thereof, and the like). In certain embodiments, the composition is dispensed via a metered dose device.

In preferred embodiments, the polymeric film forming spray formulations of the invention provide a modified release of the active agent(s) when applied or sprayed onto an environment of use (e.g., human skin). The permeation or release of the drug following topical application from the invention is characterized by a biphasic release profile, the first phase comprising an immediate release of drug from the drug-solvent micro-droplet that is facilitated by the small (e.g., approximately 50 microns) spray droplet size and a subsequent second controlled release phase comprising release of drug from the bio-adhesive film formed, upon evaporation of the solvent system.

The polymeric film forming topical spray formulation of the invention, by virtue of and dependent upon the choice and amount of hydrophilic polymer and the use of a hydroalcoholic solvent, has been found to provide an initial release of drug once the topical spray is applied (e.g., sprayed) onto a surface (e.g., human skin) followed by a lag during which less drug is released over a period of time. The duration of the lag time may be varied by altering the polymer composition and/or the amount of polymer and/or the choice of permeation enhancer and the form of the active agent (e.g., base or salt form, and choice of salt).

The polymeric film forming topical spray formulation provides a biphasic release of the active agent(s) in which a first portion of the active agent(s) is released either immediately or after a short time delay to provide a first peak maximum concentration at the site (for a topical drug) or in blood plasma (for a systemic treatment/drug) which occurs from 0.25 hours to 1.5 hours after application of the topical spray on the skin, and a second portion of the active agent(s) released after a lag time to provide a second peak maximum concentration at 4 hours to 12 hours after application of the topical spray on the skin.

In certain embodiments of the invention, the topical spray formulation provides a unit dose (e.g., comprising from about 1 to about 20 sprays) wherein 10% of the spray droplets in the unit dose have a mean diameter of 26 µm ± 20 µm, 50% of the spray droplets in the unit dose have a mean diameter of 55 µm ± 20 µm, and 90% of the spray droplets in the unit dose have a mean diameter of 116 µm ± 40 µm. It is believed that the smallest spray droplets provide the initial quick (first) peak concentration and the drug may even be absorbed/released at the site of application prior to the setting of the film. Thereafter, the film sets and there is a delay until the second peak concentration occurs.

The drug is supersaturated in the formulation. It is believed that the closer to supersaturation the drug is in the formulation, the more permeation is obtained when the drug is administered, e.g., by spraying onto human skin.

In a preferred embodiment of the present invention, the active ingredient is a local anesthetic in base or salt form, e.g., bupivacaine hydrochloride, and the composition in operation delivers the active ingredient in a bimodal or pulsed manner.

In another preferred embodiment of the present invention, the active ingredient is methylphenidate and the composition in operation delivers the active ingredient in a bimodal or pulsed manner. Such a composition in operation produces a plasma profile which substantially mimics that obtained by the sequential administration of two IR doses as, for instance, in a typical methylphenidate treatment regime.

In the present invention micro-emulsion, nano-emulsion and/or solid lipid nanoparticles can be suspended and or dispersed in the polymeric film forming solution for dermal spray delivery of pharmaceutical active ingredients. Micro-emulsions are system well known for their long-term stability and ease of preparation. They are stabilized by an interfacial film of surfactants, usually in combination with co-surfactants such as short chain alcohols. Micro-emulsions offer several advantages for improving local delivery and efficiency of drugs through a high solubilization capacity, increased thermodynamic activity and a reduction of the diffusional barrier of the stratum corneum. In the present invention, micro-emulsion and nano-emulsion systems are used to increase the permeability of drug (e.g., bupivacaine salts and base) through the skin after spray administration by a mechanical pump.

The polymeric solution containing an active agent(s) is preferably sprayed (e.g. via a mechanical sprayer) as microdroplets and nanodroplets onto the skin of a mammal (e.g., a human subject or a human patient). In certain preferred embodiments, a unit dose of the active agent(s) is sprayed using a metered pump delivering from about 40 ul to about 350 ul volume per spray. Once the solvent evaporates, the film sets as a non-contiguous, highly nano- or micro-porous film. Each micro- and nano- droplet may be considered to act as a separate drug delivery system.

The polymeric film forming spray formulation can be used with any pharmaceutical active ingredient in the salt or base form or in a combination of two or more active ingredients that is stable on mixing with polymer and other excipients for effective topical drug delivery.

The present invention is a multi-dose and or unit dose of a topical spray formulation, this formulation comprising liquid droplets of an active agent(s) and a pharmaceutically acceptable solvent carrier. The droplets preferably have a size distribution of from about 1 micron to about 1000 microns, and in certain preferred embodiments from about 5 microns to about 500 microns in diameter. In certain preferred embodiments, the film does not set (dry) on the applied surface (e.g., human skin) as a contiguous sheet, but instead sets as a breathable, microporous (and nanoporous) film. The drug(s) is absorbed slowly at the site of application as the drug leaches into the skin from the film. It is hypothesized that each microdroplet/nanodroplet of the topical spray acts as a separate drug delivery system on the skin.

In certain embodiments, the invention is directed to a unit dose of a topical spray pharmaceutical formulation comprising a polymeric solution, emulsion or suspension of povidone in an amount from 2.5 to 20% by weight, a therapeutically effective amount of a supersaturated active agent, and a pharmaceutically acceptable permeation enhancer dispersed in a pharmaceutically acceptable hydroalcoholic solvent, the unit dose comprising a plurality of spray droplets, wherein 10% of the spray droplets in the unit dose have a mean diameter of 26 µm ± 20 µm, 50% of the spray droplets in the unit dose have a mean diameter of 55 µm ± 20 µm, and 90% of the spray droplets in the unit dose have a mean diameter of 116 µm ± 40 µm, the unit dose topical spray provides a film surface area from 1cm² to 40 cm² per spray and sets as a microporous, breathable and bioadhesive film when the hydroalcoholic solvent evaporates and provides a biphasic release of the active agent. In certain preferred embodiments, 10% of the spray droplets in the unit dose have a mean diameter of 26 µm ± 1.82 µm, 50% of the spray droplets in the unit dose have a mean diameter of 55 µm ± 2.39 µm, and 90% of the spray droplets in the unit dose have a mean diameter of 116 µm ± 4.9 µm. In certain embodiments, the first peak concentration occurs at from 0.17 to 0.67 hours after the unit dose is sprayed onto the human subject, and the second peak concentration occurs at from 4 to 24 hours after the unit dose is sprayed onto the human subject. Preferably, the second peak concentration occurs at from 9 to 24 hours after the unit dose is sprayed onto the human subject. In certain preferred embodiments where the drug is bupivacaine hydrochloride, the first peak concentration occurs at from 0.17 to 0.67 hours after the unit dose is sprayed onto the human subject, and the second peak concentration occurs at from 4 to 24 hours after the unit dose is sprayed onto the human subject. Preferably the second peak concentration occurs at from 9 to 24 hours after the unit dose is sprayed onto the human subject. In certain preferred embodiments, the bupivacaine hydrochloride is present in the unit dose in an amount from 0.5 to 40 mg, more preferably from 0.5 to 20 mg. The bupivacaine hydrochloride is present in a supersaturated solution in the formulation. It has been found that this occurs, e.g., when the amount of bupivacaine in the unit dose is at least about 12 mg per spray.

In certain preferred embodiments, the unit dose provides a first peak concentration from 29 pg/ml to 380 pg/ml bupivacaine, and a second peak plasma concentration from 864 pg/ml to 3463 pg/ml bupivacaine.

In certain preferred embodiments, the film forming topical spray formulation of the invention comprises from 2.5% to 20% hydrophilic film forming polymer which is povidone, from 0.05% to 35% active agent(s), from 0.05% to 50% permeation enhancer, and from 0.01% to 30% optional additional pharmaceutically acceptable excipients (as described herein), in a hydroalcoholic solvent. In certain most preferred embodiments, the film forming topical spray formulation comprises from 2.5% to 7.5% by weight hydrophilic film forming polymer. In certain preferred embodiments, the film forming topical spray formulation comprises from 1% to 15% by weight permeation enhancer(s). In certain preferred embodiments, the film forming topical spray formulation comprises from 2% to 12% by weight permeation enhancer(s), and in certain embodiments most preferably from 2.5% to 10% permeation enhancer.

In certain embodiments, the film forming topical spray formulation further comprises from 1% to 35% hydrophobic polymer.

In certain preferred embodiments, the film forming topical spray formulation of the invention consists of from 2.5% to 20% hydrophilic film forming polymer which is povidone, from 0.75% to 20% active agent(s), from 2.5% to 20% permeation enhancer, and from 0.05% to 20% optional additional pharmaceutically acceptable excipients (as described herein), in a pharmaceutically acceptable solvent.

The solvent concentration is from 20 to 99%, by weight. In certain preferred embodiments, the solvent is a hydroalcoholic solvent as described herein.

The surface area to be covered by the film forming topical spray formulation will vary depending upon the area to be treated and the active agent(s), and the intended effect (e.g., topical versus systemic). In general, the film surface area is preferably from about 1 cm² to about 50 cm² per spray, and in certain embodiments more preferably from about 1 cm² to about 20 cm² per spray. In general, the number of sprays per application can vary between 1 to about 7 or between 1 to about 20 sprays. The droplet size distribution in each spray of the topical spray formulation (e.g., via a pump spray) will provide micro- and nano-droplets comprising the active agent(s), hydrophilic polymer and permeation enhancer in a (preferably) hydroalcoholic solvent. The formulation can be in solution, suspension, gel or emulsion form.

In certain preferred embodiments, the film is transparent. However, in other embodiments, the topical spray solution is colored, either due to the ingredients themselves or via the addition of a pharmaceutically acceptable colorant which may be applied to the skin and/or systemically absorbed.

In certain embodiments, the formulation may be sterilized in any pharmaceutically acceptable manner known to those skilled in the art.

### Film-Forming Polymer

The film forming polymer includes an effective amount of a hydrophilic polymer to provide the desired release of active agent as described herein. The formulation comprises 2.5 to 20% by weight polyvinyl pyrolidone (also known as PVP, povidone or copovidone). Other hydrophilic polymers may be present and include polyvinyl alcohol, polyvinyl acetate, water soluble gums, water soluble celluloses (e.g., hydroxypropylmethylcellulose, hydroxypropylcellulose, hydroxyethylcellulose and the like), dextrans, hyaluronic acid, cyclodextrins, polysaccharide polymers, polyvinyl caprolactam - polyvinyl acetate - polyethylene glycol graft copolymer and combinations of any of the foregoing. In certain embodiments, the film forming polymer consists of hydrophilic polymer(s) only.

The film forming polymer comprises povidone in an amount from 2.5% to 20% of the topical spray formulation.

In certain embodiments, part of the hydrophilic film forming polymer comprises hydrocolloids not limited to, synthetic gums or natural gums such as plant exudates (gum arabic, ghatti, karaya, and tragacanth); plant seed gums (guar, locust bean and acacia), seaweed extracts (agar, algin, alginate salts and carrageenin), cereal gums (starches and modified starches), fermentation or microbial gums (dextran and xanthan gum), biosynthetic gums, gelatins, pectin, casein, welan gum, gellan, rhamsan gum, synthetic gums (low methoxyl pectin, propyleneglycol alginates, carboxymethyl locust bean gum and carboxymethyl guar gum), pullulan, other water-swellable or hydratable hydrocolloid gums known to those skilled in the art, and the like. The term hydrocolloid gum is used regardless of the state of hydration. The hydrocolloid gum can provide suitable viscosity to provide a flowable formulation that may be utilized in conjunction with the various embodiments of the invention described herein. The hydrocolloid gum utilized in the formulation of the invention may also comprise a combination of gums.

The formulation comprises a drug crystal precipitation inhibiting agent which prevents or substantially prevents the drug(s) included in the formulation from precipitating. The hydrophilic film forming polymer and the drug crystal precipitation inhibiting agent are comprised of the same agent which is povidone.

In certain embodiments, up to about 35% of the film forming polymer may be comprised of a hydrophobic polymer. Suitable hydrophobic polymers include acrylate copolymers, methacrylic polymers and copolymers, acrylate/octylacrylamide copolymer, methyl cellulose, ethyl cellulose, methacrylic polymer, Polyurethane-14 and AMP-acrylates copolymer, Poly(butyl methacrylate, (2-dimethylaminoethyl)methacrylate, methyl methacrylate) 1:2:1, Poly(ethyl acrylate, methyl methacrylate) 2:1, Poly(ethyl acrylate, (2-trimethylaminoethyl)methacrylate, methyl methacrylate) 1:0.2:2 chloride, Poly(methacrylic acid, methyl methacrylate) 1:2, Polyisobutylene, silicon gum, silicon dioxide, microcrystalline cellulose, sodium carboxyl cellulose, and the like.

In certain preferred embodiments where the hydrophobic polymer comprises acrylic polymers or copolymers, methacrylic polymers and copolymers, including ethoxyethyl methacrylates, cynaoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolymer, poly(methacrylic acid anhydride), and glycidyl methacrylate copolymers. In certain embodiments, the acrylic polymer is comprised of one or more ammonia methacrylate copolymers. Ammonia methacrylate copolymers are well known in the art, and are described in NF XVII as fully polymerized copolymers of acrylic and methacrylic acid esters with a low content of quaternary ammonium groups. Preferred film-formers include a non-ionic copolymer of methyl methacrylate and butyl methacrylate (Plastoid B®), a copolymer of dimethylamine ethyl methacrylate and a neutral methacrylic acid ester (Eudragit E100®), ammonio methacrylate copolymer type B (Eudragit RS®), ammonio methacrylate copolymer type A (Eudragit RL®), methacrylic acid copolymer type A (Eudragit L100®), methacrylic acid copolymer type B (Eudragit S100®), and the like.

The hydrophobic or hydrophobic polymers may be selected from cellulose and cellulose derivatives (such as the cellulose ethers and esters mentioned previously), starches or modified starches, as well as other pharmaceutically acceptable hydrophobic materials known to those skilled in the art. An example of a preferred hydrophobic cellulosic material is ethylcellulose. Other useful cellulose derivatives (including but not limited to hydroxymethylcellulose, carboxymethylcellulose, hydroxyethylcellulose, hydroxymethylcellulose, starch, hydroxypropylmethylcellulose, hydroxypropylcellulose, methylcellulose, hydroxypropylmethylcellulose), carboxyvinyl polymers, poly-1,4-glucans, e.g., starch glycogen, amylose, amylopectin, carboxyvinyl polymers, combinations or mixtures thereof, and the like. Useful water-soluble derivatives of poly-1,4-glucans include hydrolyzed amylopectin, hydroxyalkyl derivatives of hydrolyzed amylopectin such as hydroxyethyl starch (HES), hydroxyethyl amylose, dialdehyde starch, and the like.

Suitable hydrophilic polymers include but are not limited to poly(acrylic acid), hydroxylpropyl methyl cellulose, polyethylene glycol, and polyethylene oxide.

In certain embodiments, the film-forming polymer can provide thixotropic behavior in use. For example, the film-forming polymer may be an amidic derivative of a carboxymethyl cellulose based thixotropic gel, and forms a homogenous three-dimensional scaffold, which maintains the thixotropic property of the (linear) polysaccharide. Amphiphilic derivatives of sodium alginate, prepared by chemical covalent binding of long alkyl chains on to the polysaccharide backbone via ester functions, form strong hydrogels in aqueous solutions with shear-thinning and thixotropic behavior. Such a hydrogel was used for the carrier or encapsulation of pharmaceutical active ingredients. In such embodiments, the thixotropic gel spray was prepared by adding the film forming polymer and thixotropic agent to the solvent and stirring the solution to ensure complete hydration and or dissolution of the polymers. The solvent used was water, hydro-alcoholic and Ethanol (95 %) for preparations. Having obtained a suspended hydrated polymer gel drug, volatile solvent, permeation enhancers were added and other optional excipients. After addition of all excipients the solution was stirred to ensure complete dissolution of drug and excipients before use. The formulations were stored in glass vials sealed tightly with a cap or spray pump.

The initial permeation rate of the film forming topical spray formulation of the invention increases with decreasing polymer concentration. However, in order to obtain better film properties, a polymer concentration in certain embodiments of 5% or greater is preferred.

### Solvents

The preferred solvent for the polymeric film forming topical spray formulation includes ethanol, isopropyl alcohol, acetone, n-butanol, methylene chloride, methylene dimethyl ether, water, hydroalcoholic system alone and or in combination of two or more solvents. The solvent concentration in the topical spray formulation is between 20% to 99% of the formulation. Preferably, the solvent is a hydroalcoholic solvent.

### Permeation Enhancers

The polymeric film forming topical spray formulation further comprises an effective amount one or more permeation enhancers which allows a sufficient amount of the dose of the drug(s) to permeate through the skin. Preferred permeation enhancers include isopropyl myristate, Oleic acid, Capric acid, Lauric acid, Lauric acid, pharmaceutically acceptable glycol derivatives (e.g., propylene glycol, diethylene glycol monoethyl ether), methyloleate, lysophosphatidylcholine, phosphatidylcholine, aprotinin, azone, cyclodextrin, dextran sulfate, menthol, polysorbate 80, sulfoxides and various alkyl glycosides, urea, ethanol, caprylic alcohol, oleyl alcohol, n-methyl-2-pyrrolidone, sodium lauryl sulfate, isostearic acid, oleth-2, polyethylene glycol, polyoxyl cetostearyl ether, polyoxyl oleyl ether, polyoxyl lauryl ether, polyoxyl stearyl ether, benzyl alcohol, mixtures of any of the foregoing, and the like. In certain preferred embodiments, the permeation enhancer is selected from isopropyl myristate, oleth-2, oleic acid, 2-pyrolidone, isostearic acid, oleyly alcohol, polysorbate 80, polyethylene glycol 600, and mixtures of any of the foregoing.

In embodiments where the permeation enhancer is polyethylene glycol, all grades of polyethylene glycol are contemplated for use in preparation of the local anesthetic stock. Polyethylene glycol is available in many different grades having varying molecular weights. For example, polyethylene glycol is available as PEG 200; PEG 300; PEG 400; PEG 540 (blend); PEG 600; PEG 900; PEG 1000; PEG 1450; PEG 1540; PEG 2000; PEG 3000; PEG 3350; PEG 4000; PEG 4600 and PEG 8000. In certain embodiments the polyethylene glycol is preferably PEG 300.

In certain embodiments, the base may be a polysorbate. Polysorbates are nonionic surfactants of sorbitan esters. Polysorbates useful in the present invention include, but are not limited to polysorbate 20, polysorbate 40, polysorbate 60, polysorbate 80 (Tween 80) and any combinations or mixtures thereof. In certain preferred embodiments, polysorbate 80 may be utilized as the pharmaceutically acceptable permeation enhancer.

It is recognized that the permeation enhancer may also act as a plasticizer. In this regard, the polymeric film forming topical spray formulation may further comprise one or more plasticizers. Suitable plasticizers include but are not limited to propylene glycol, polyethylene glycol, citrate esters, dimethyl isosorbide, castor oil, and combinations of any of the foregoing.

In the case of thinner film thickness, permeation enhancer concentration becomes more significant for the initial permeation rate of active through membrane or skin. The thinner film thickness alternatively represents film spread over a larger surface area on human skin after spray administration.

### Optional Excipients

In addition to the active agent(s) (e.g., local anesthetic), the polymeric film forming topical spray formulation may additionally include physiologically acceptable components such as sodium chloride and like materials conventionally used to achieve isotonicity with typical body fluids, pH buffers to establish a physiologically compatible pH range and to enhance the solubility of the anesthetic present, vasoconstrictors such as epinephrine, preservatives, stabilizers and antioxidants and the like.

In certain other embodiments, an additional surfactant (co-surfactant) and/or buffering agent can preferably be combined with one or more of the pharmaceutically acceptable vehicles previously described herein so that the surfactant and/or buffering agent maintains the product at an optimal pH for stability. The surfactant and/or buffering agent may also prevent the initial stinging or burning discomfort associated with administration of the active agent on the skin (e.g, local anesthetic).

In certain other embodiments, an additional anti-oxidant and/or stabilizing agent can preferably be combined with one or more of the pharmaceutically acceptable vehicles previously described herein so that the anti-oxidant and/or stabilizing agent maintains the drug product at an optimal impurity level for stability. The anti-oxidant and/or stabilizing agent also prevents the initial degradation of active agent during the manufacturing process. The anti-oxidant may be selected, e.g., from ascorbic acid, EDTA, trolamine, tocopherol, propyl galate, sodium sulfite and mixtures of any of the foregoing.

The formulation of the invention may contain preservatives to prevent microbial growth. Suitable preservatives for use in the present invention include, but are not limited to benzoic acid, boric acid, p-hydroxybenzoates, phenols, chlorinated phenolic compounds, alcohols, quarternary compounds, mercurials, mixtures of the foregoing and the like.

The composition may further comprise one or more additional components selected from the group consisting of solubilizers, plasticizers, and water soluble additives.

Preferred plasticizers include triethyl citrate, dimethyl isosorbide, acetyltributyl citrate, castor oil, propylene glycol, and polyethylene glycol, or any two or more of the above in combination.

### Viscosity of the Formulation

In most applications, optimal viscosities of the system of the invention will range from about 10 to about 2,000,000 centipoise, preferably from about 0.3 to 1000 centipoise, and more preferably from about 0.5 to about 100 centipoise, at 37°C. While the benefit of the invention is realized over a broad range of elevated viscosities, the optimal viscosities will be different for different applications. The desired viscosity for any given formulation or use may vary, for example, according to the preference of the physician, the manner of application and type of applicator used, the amount of formulation needed, the area to which the formulation is to be applied, and similar considerations.

### Active Agents

The amount of drug permeating through the membrane is directly proportional to concentration of active (drug) in the formulation, with the greater amount of drug concentration in the formulation leading to greater cumulative drug permeation.

In certain preferred embodiments of the invention, the active agent comprises a local anesthetic. Examples of local anesthetic agents useful in the gel formulations of the invention include amide type local anesthetics, such as mepivacaine, lidocaine, mepivacaine, etidocaine and prilocaine; ester type local anesthetics, such as procaine, chloroprocaine, and tetracaine; and antihistamine-like anesthetics, such as benadryl. These anesthetics can be present in the anesthetic pharmaceutical combination alone or as a mixture of two or more thereof. Thus, examples of useful local anesthetics are lidocaine, bupivacaine, dibucaine, tetracaine, etidocaine, mepivacaine, ropivacaine, benzocaine, ambucaine, amylocaine, butamben, 2-chloroprocaine, cyclomethycaine, ethyl aminobenzoate, euprocin, levoxadrol, orthocaine, piperocaine and parethoxycaine. In certain preferred embodiments, the local anesthetic is bupivacaine, ropivacaine, dibucaine, procaine, chloroprocaine, prilocaine, mepivacaine, etidocaine, tetracaine, lidocaine, and xylocaine, or mixtures thereof. In certain preferred embodiments, the local anesthetic is lidocaine, bupivacaine, levo-bupivacaine, ropivacaine, tetracaine, mepivacaine, prilocaine, benzocaine, editocaine, or combinations of any of the foregoing. The phrase "local anesthetic" also can include drugs of a different class than those traditionally associated with local anesthetic properties, such as morphine, fentanyl, and agents which, for example, can provide regional blockade of nociceptive pathways (afferent and/or efferent). Other compounds which may be used as a local anesthetic in the gel formulations of the invention include antihistamine-like anesthetics, such as benadryl. Phenol may also be used as the local anesthetic. Those skilled in the art will recognize other agents which have been recognized to possess local anesthetic properties, such as the substituted piperidines and pyrollidines described in U.S. Patent No. 4,302,465 (Aberg, et al.) and the aminoindane piperidine compounds described in U.S. Patent No. 6,413,987 (Aberg, et al.). The term local anesthetic is also deemed for purposes of the present invention to encompass the local anesthetic base or a pharmaceutically acceptable salt, polymorph, complex or pro-drug thereof. Many other examples of both drugs and local anesthetics will be readily apparent to those skilled in the art, and are considered to be encompassed by this disclosure and appended claims.

The local anesthetic can be in the form of a salt, for example, the hydrochloride, bromide, acetate, citrate, carbonate, sulfate or phosphate. In certain embodiments, the local anesthetic agent is in the form of a free base. Local anesthetics can be in the form of a salt, for example, the hydrochloride, bromide, acetate, citrate, carbonate or sulfate, or in the form of a free base. Many of the local anesthetics are conventionally used in the form of their acid addition salts, as this provides solubility in aqueous injection media. In certain embodiments of the invention, it is desirable to use the local anesthetics in free base form, or with only a small proportion of the acid addition salt of the local anesthetic present (addition of small quantities of the acid addition salt may provide enhanced release if desired). The free base generally provides a slower initial release and avoids an early "dumping" of the local anesthetic at the injection site. Preferred local anesthetic agents include, e.g., bupivacaine, ropivacaine or lidocaine. For bupivacaine, the free base provides a slower initial release and avoids an early "dumping" of the local anesthetic at the site of administration. Also, it has been found that the formulations provide dose proportionality with the bupivacaine salt (i.e., bupivacaine hydrochloride), but not with the base form. Other local anesthetics may act differently.

In certain preferred embodiments, the dose of local anesthetic contained in a unit dose is from about from 0.01mg to 50 mg bupivacaine. In other preferred embodiments, the dose is from 0.1 mg to 20 mg bupivacaine, and preferably from 1 mg to 10 mg or to 20mg bupivacaine.

In other embodiments, the active agent is an anesthetic such as a barbiturate (e.g., amobarbital, methohexital, thiamylal, thiopental), a benzodiazepine (e.g., diazepam, lorazepam, midazolam), or etomidate, ketamine, or propofol.

In certain embodiments of the invention, the film forming topical spray formulation provides a systemic effect when applied or sprayed onto an environment of use (e.g., human skin). Any active ingredient (drug) for which it is useful to combine the advantages of a pulsatile plasma profile may be used in practice of the present invention, including but not limited to active agents whose pharmacological and/or therapeutic effects benefit from having a wash-out period between plasma concentration peaks, such as those active ingredients susceptible to the development of patient tolerance. Example active ingredients (drugs) include but are not limited to peptides or proteins, hormones, analgesics, anti-migraine agents, anti-coagulant agents, narcotic antagonists, chelating agents, anti-anginal agents, chemotherapy agents, sedatives, anti-neoplastics, prostaglandins and antidiuretic agents, drug compounds acting on the central nervous system such as cerebral (central nervous system) stimulants, for example methylphenidate; pain management active ingredients; alkaloids such as opiates, for example morphine; cardiovascular drugs, such as nitrates; and agents for treating rheumatic conditions. It should be further appreciated that the present invention may be used to deliver a number of drugs including, but not limited to, peptides, proteins or hormones such as insulin, calcitonin, calcitonin gene regulating protein, growth factor (somatomedins), luteinizing hormone releasing hormone (LHRH), tissue plasminogen activator (TPA), growth hormone releasing hormone (GHRH), oxytocin, estradiol, growth hormones, leuprolide acetate, factor VIII, interleukins such as interleukin-2, and analogues thereof; analgesics such as fentanyl, sufentanil, butorphanol, buprenorphine, levorphanol, morphine, hydromorphone, hydrocodone, oxymorphone, methadone, lidocaine, bupivacaine, diclofenac, naproxen, paverin, and analogues thereof; anti-migraine agents such as sumatriptan, ergot alkaloids, and analogues thereof; anti-coagulant agents such as heparin, hirudin, and analogues thereof; anti-emetic agents such as scopolamine, ondansetron, domperidone, metoclopramide, and analogues thereof; cardiovascular agents, anti-hypertensive agents and vasodilators such as diltiazem, clonidine, nifedipine, verapamil, isosorbide-5-mononitrate, organic nitrates, agents used in treatment of heart disorders, and analogues thereof; sedatives such as benzodiazepines, phenothiozines, and analogues thereof; chelating agents such as deferoxamine, and analogues thereof; anti-diuretic agents such as desmopressin, vasopressin, and analogues thereof; anti-anginal agents such as nitroglycerine, and analogues thereof; anti-neoplastics such as fluorouracil, bleomycin, and analogues thereof; prostaglandins and analogues thereof; and chemotherapy agents such as vincristine, and analogues thereof.

In certain preferred embodiments, the active agent may be an opioid analgesic(s), including but not limited to buprenorphine, morphine, oxycodone, hydromorphone, codeine, oxymorphone, fentanyl, sufentanyl, tramadol, and hydrocodone.

In other preferred embodiments, the active agent may be a non-opioid analgesic including but limited to non-steroidal anti-inflammatory agents such as ibuprofen, naproxen, diclofenac, naproxen, ketoprofen, ketorolac, and the like.

The active agent may also be an anti-bacterial agent including, but not limited to mupirocin, bacitracin, neomycin, penicillins, cephalosporins, vancomycin, bacitracin, cephalosporins, polymxyins, amikacin, doxycycline, nystatin, amphotericin-B, tetracyclines, chloramphenicol, erythromycin, minocycline, gentamicin, neomycin, streptomycin, kanamycin, gentamicin, tobramycin, clindamycin, rifampin, nalidixic acid, flucytosine, griseofulvin, clindamycin, doxycycline, mixtures of any of the foregoing, and the like.

The active agent may also be an antiviral agent including but not limited to vidarabine, acyclovir, ribavirin, amantadine hydrochloride, interferons, dideoxyuridine, mixtures of any of the foregoing and the like.

The active agent may also be an antifungal agent including but not limited to butoconazole, tetraconazole, nystatin, miconazole, tolnaftate, undecyclic acid and its salts, mixtures of the foregoing and the like.

The active agent may alternatively be an antiparasitic agent including but not limited to quinacrine, chloroquine, quinine, mixtures of the foregoing and the like.

The active agent may also be a steroidal anti-inflammatory agents including but not limited to hydrocortisone, prednisone, fludrocortisone, triamcinolone, dexamethasone, betamethasone, desoximetasone, fluticasone mixtures of the foregoing and the like.

The active agent may also be an antihistamine (H2 antagonist) including, but not limited to diphenhydramine, chlorpheneramine, chlorcyclizine, promethazine, cimetidine, terfenadine, mixtures of the foregoing and the like.

Other preferred pharmaceutical active agents (drugs) which can be incorporated into the formulations of the invention include epinephrine, clonidine, methylphenidate, nicotine, nitroglycerin, oxybutynin, rotigotine, selegiline, scopolamine, ciclopirox, misoprostol, hyoscyamine, atropine, silver sulfadiazine, sulfanilamide, clotrimazole, terbinafine, ketoconazole, acyclovir, minoxidil, tretinoin, azelaic acid, benzoyl peroxide, capsaicin, clobetasol, desonide, miconazole, tacrolimus, salicylic acid, terbenafine, clobetasol, sumatriptan, zolmitriptan, triamcinolone, zolpidem, rivastigmine, piroxicam, amytriptylene, meloxicam, dalfampridine, spiro-oxindole compounds, gabapentin, ketamine, docosanol, pirfenidone, isotretinoin, and papaya enzymes. The active agents (drugs) can be in the base form, or can be pharmaceutically acceptable salts, complexes or derivatives of the active agent.

The formulations of the invention can include two or more of the above-mentioned ingredients (drugs) or pharmaceutically acceptable salts, complexes or derivatives thereof, as well.

In certain preferred embodiments, the active agent(s) (drug(s)) is a steroid, such as estrogen, estradiol, norethindrone, levonorgestrel, ethinylestradiol, norelgestromin, testosterone, and mixtures thereof.

In certain preferred embodiments, the active agent(s) (drug(s)) is a combination of therapeutically effective amounts of a local anesthetic (e.g., bupivacaine) and ketamine and or amitriptyline for use in treating neuropathic pain. In certain embodiments, the active agent comprises a combination of bupivacaine hydrochloride and a second active agent selected from the group consisting of ketamine, amitriptyline, and combinations thereof.

The composition contains preferably up to about 30% of the at least one medicament (drug), more preferably up to about 10% of the at least one drug and most preferably up to about 5% of the at least one drug.

The compositions are preferably in a form suitable for application by spraying from an aerosol or pump spray container.

### Treatment of Pain

Neuropathic pain is a persistent pain condition that develops secondary to nerve injury. The two most common types of peripheral neuropathic pain are post-herpetic neuralgia (PHN) and painful peripheral diabetic neuropathy (PDN). PHN is a serious complication of herpes zoster or shingles, which occurs in some patients secondary to reactivation of varicella-zoster virus residing in the dorsal root ganglia of individuals following primary infection (chicken pox) often in childhood. The main symptoms experienced during the acute period of herpes zoster infection are pain and skin rash. The pain that emerges subsequent to the healing of rash and which persists for more than three months is known as PHN. The incidence of PHN among herpes zoster patients is approximately 10-34% with a higher incidence in elderly patients (>60 years), 50% of whom are likely to develop PHN

At present, there are no treatments that can reverse 'neuropathic nerve injury'. Therefore treatments are strictly palliative, targeted to the provision of symptomatic pain relief with the treatment goal to reduce pain to tolerable levels. The current available pharmacological treatments for the treatment of symptomatic relief of PHN include antidepressants, serotonin noradrenaline reuptake inhibitors (SNRIs), selective serotonin reuptake inhibitors (SSRIs), anti-epileptics, opioids, N-Methyl D-Aspartate (NMDA) receptor antagonists and topical anesthetics like lidocaine, bupivacaine, ropivacaine. Lidocaine 5% patch is a topical agent that has been approved by the FDA for symptomatic relief of PHN. The primary mechanism of action is through lidocaine inhibiting voltage-gated Na+ channels in damaged peripheral nerves that have developed pathological spontaneous activity, which is thought to be associated with injury-induced increased expression of Na+ channels.

Lidocaine (xylocaine) was introduced as a local anesthetic in 1948. Local anesthetics act by preventing the generation and conduction of nerve impulse. Their primary site of action is the cell membrane. They block conduction by decreasing or preventing the large transient increase in the permeability of excitable membranes to Na+ that normally is produced by a slight depolarization of the membrane. This action of local anesthetics is due to their direct interaction with voltage gated Na+ channels. As the anesthetic action progressively develops in a nerve, the threshold for electrical excitability gradually increases, the rate of rise of the action potential declines, impulse conduction slows, and the safety factor for conduction decreases; these factors decrease the probability of propagation of the action potential, and nerve conduction fails. Bupivacaine, a longer acting variant of lidocaine is a preferred local analgesic.

The degree of block produced by a given concentration of local anesthetic depends on how the nerve has been stimulated and on its resting membrane potential. Thus, a resting nerve is much less sensitive to a local anesthetic than is one that is repetitively stimulated; higher frequency of stimulation and more positive membrane potential cause a greater degree of anesthetic block. These frequency and voltage dependent effects of local anesthetics occur because the local anesthetic molecule in its charged form gains access to its binding site within the pore only when the Na+ channel is in an open state and because the local anesthetic molecule binds more tightly to and stabilizes the inactivated state of the Na+ channel. Local anesthetics exhibit these properties to different extents depending on their pKa, lipid solubility and molecular size.

Lidoderm patch is cumbersome to use. It is supplied as a 10 cm X 14 cm patch. The patient is instructed to apply three patches to the most painful area once for up to 12 hours. Per the package insert, the site of patch application may develop erythema, edema, bruising, papules, vesicles, discoloration, depigmentation, burning sensation, pruritus and abnormal sensation which is reversed upon patch removal. The patch is not patient friendly more so when the pain presents itself outside the upper body and trunk area specifically in the facial area (myofascial pain). It is anticipated that the present invention, which is an easy to use proprietary dermal spray formulation to overcome many of the disadvantages of patch application while still providing pain relief in patients with PHN. A topical spray formulation would provide application convenience.

Accordingly in certain preferred embodiments, the topical spray formulation of the invention is used for treating general pain, neuropathic pain neuropathic pain (e.g., erythromelalgia, post-herpetic neuralgia (PHN), fibromyalgia and/or complex regional pain syndrome (CRPS), among others), postoperative pain, sports pain, osteoporosis pain, pain resulting from cosmetic procedures, dental pain, wound pain and burn pain.

In certain preferred embodiments, the topical spray formulation of the invention is used to reduce pain during the treatment of or amelioration of symptoms of any one or more of the following diseases which cause neuropathic pain or which have a neuropathic pain component: Abdominal Wall Defect, Abdominal Migraine, Achondrogenesis, Achondrogenesis Type IV, Achondrogenesis Type III, Achondroplasia, Achondroplasia Tarda, Achondroplastic Dwarfism, Acquired hnmunodeficiency Syndrome (AIDS), Acute Intermittant Porphyria, Acute Porphyrias, Acute Shoulder Neuritis, Acute Toxic Epidermolysis, Adiposa Dolorosa, Adrenal Neoplasm, Adrenomyeloneuropathy, Adult Dermatomyositis, Amyotrophic Lateral Sclerosis, Amyotrophic Lateral Sclerosis-Polyglucosan Bodies, AN, AN 1, AN 2, Anal Rectal Malformations, Anal Stenosis, Arachnitis, Arachnoiditis Ossificans, Arachnoiditis, Arteritis Giant Cell, Arthritis, Arthritis Urethritica, Ascending Paralysis, Astrocytoma Grade I (Benign), Astrocytoma Grade II (Benign), Athetoid Cerebral Palsy, Barrett Esophagus, Barrett Ulcer, Benign Tumors of the Central Nervous System, Bone Tumor-Epidermoid Cyst-Polyposis, Brachial Neuritis, Brachial Neuritis Syndrome, Brachial Plexus Neuritis, Brachial-Plexus-Neuropathy, Brachiocephalic Ischemia, Brain Tumors, Brain Tumors Benign, Brain Tumors Malignant, Brittle Bone Disease, Bullosa Hereditaria, Bullous CIE, Bullous Congenital Ichthyosiform Erythroderma, Bullous Ichthyosis, Bullous Pemphigoid, Burkitt's Lymphoma, Burkitt's Lymphoma African type, Burkitt's Lymphoma Non-african type, Calcaneal Valgus, Calcaneovalgus, Cavernous Lymphangioma, Cavernous Malformations, Central Form Neurofibromatosis, Cervical Spinal Stenosis, Cervical Vertebral Fusion, Charcot's Disease, Charcot-Marie-Tooth, Charcot-Marie-Tooth Disease, Charcot-Marie-Tooth Disease Variant, Charcot-Marie-Tooth-Roussy-Levy Disease, Childhood Dermatomyositis, Chondrodysplasia Punctata, Chondrodystrophia Calcificans Congenita, Chondrodystrophia Fetalis, Chondrodystrophic Myotonia, Chondrodystrophy, Chondrodystrophy with Clubfeet, Chondrodystrophy Epiphyseal, Chondrodystrophy Hyperplastic Form, Chondroectodermal Dysplasias, Chondrogenesis Imperfecta, Chondrohystrophia, Chondroosteodystrophy, Chronic Adhesive Arachnoiditis, Chronic Idiopathic Polyneuritis (CIP), Chronic Inflammatory Demyelinating Polyneuropathy, Chronic Inflammatory Demyelinating Polyradiculoneuropathy, Cicatricial Pemphigoid, Complex Regional Pain Syndrome, Congenital Cervical Synostosis, Congenital Dysmyelinating Neuropathy, Congenital Hypomyelinating Polyneuropathy, Congenital Hypomyelination Neuropathy, Congenital Hypomyelination, Congenital Hypomyelination (Onion Bulb) Polyneuropathy, Congenital Ichthyosiform Erythroderma, Congenital Tethered Cervical Spinal Cord Syndrome, Cranial Arteritis, Crohn's Disease, Cutaneous Porphyrias, Degenerative Lumbar Spinal Stenosis, Demyelinating Disease, Diabetes Mellitus Diabetes Insulin Dependent, Diabetes Mellitus, Diabetes Mellitus Addison's Disease Myxedema, Discoid Lupus, Discoid Lupus Erythematosus, Disseminated Lupus Erythematosus, Disseminated Neurodermatitis, Disseminated Sclerosis, EDS Kyphoscoliotic, EDS Kyphoscoliosis, EDS Mitis Type, EDS Ocular-Scoliotic, Elastosis Dystrophica Syndrome, Encephalofacial Angiomatosis, Encephalotrigeminal Angiomatosis, Enchondromatosis with Multiple Cavernous Hemangiomas, Endemic Polyneuritis, Endometriosis, Eosinophilic Fasciitis, Epidermolysis Bullosa, Epidermolysis Bullosa Acquisita, Epidermolysis Bullosa Hereditaria, Epidermolysis Bullosa Letalias, Epidermolysis Hereditaria Tarda, Epidermolytic Hyperkeratosis, Epidermolytic Hyperkeratosis (Bullous CIE), Familial Lumbar Stenosis, Familial Lymphedema Praecox, Fibromyalgia, Fibromyalgia-Fibromyositis, Fibromyositis, Fibrositis, Fibrous Ankylosis of Multiple Joints, Fibrous Dysplasia, Fragile X syndrome, Generalized Fibromatosis, Guillain-Barre Syndrome, Hemangiomatosis Chondrodystrophica, Hereditary Sensory and Autonomic Neuropathy Type I, Hereditary Sensory and Autonomic Neuropathy Type II, Hereditary Sensory and Autonomic Neuropathy Type III, Hereditary Sensory Motor Neuropathy, Hereditary Sensory Neuropathy type I, Hereditary Sensory Neuropathy Type I, Hereditary Sensory Neuropathy Type II, Hereditary Sensory Neuropathy Type III, Hereditary Sensory Radicular Neuropathy Type I, Hereditary Sensory Radicular Neuropathy Type I, Hereditary Sensory Radicular Neuropathy Type II, Herpes Zoster, Hodgkin Disease, Hodgkin's Disease, Hodgkin's Lymphoma, Hyperplastic Epidermolysis Bullosa, Hypertrophic Interstitial Neuropathy, Hypertrophic Interstitial Neuritis, Hypertrophic Interstitial Radiculoneuropathy, Hypertrophic Neuropathy of Refsum, Idiopathic Brachial Plexus Neuropathy, Idiopathic Cervical Dystonia, Juvenile (Childhood) Dermatomyositis (JDMS), Juvenile Diabetes, Juvenile Rheumatoid Arthritis, Pes Planus, Leg Ulcer, Lumbar Canal Stenosis, Lumbar Spinal Stenosis, Lumbosacral Spinal Stenosis, Lupus, Lupus, Lupus Erythematosus, Lymphangiomas, Mononeuritis Multiplex, Mononeuritis Peripheral, Mononeuropathy Peripheral, Monostotic Fibrous Dysplasia, Multiple Cartilaginous Enchondroses, Multiple Cartilaginous Exostoses, Multiple Enchondromatosis, Multiple Myeloma, Multiple Neuritis of the Shoulder Girdle, Multiple Osteochondromatosis, Multiple Peripheral Neuritis, Multiple Sclerosis, Musculoskeletal Pain Syndrome, Neuropathic Amyloidosis, Neuropathic Beriberi, Neuropathy of Brachialpelxus Syndrome, Neuropathy Hereditary Sensory Type I, Neuropathy Hereditary Sensory Type II, Nieman Pick disease Type A (acute neuronopathic form), Nieman Pick disease Type B, Nieman Pick Disease Type C (chronic neuronopathic form), Non-Scarring Epidermolysis Bullosa, Ochronotic Arthritis, Ocular Herpes, Onion-Bulb Neuropathy, Osteogenesis Imperfect, Osteogenesis Imperfecta, Osteogenesis Imperfecta Congenita, Osteogenesis Imperfecta Tarda, Peripheral Neuritis, Peripheral Neuropathy, Perthes Disease, Polyarteritis Nodosa, Polymyalgia Rheumatica, Polymyositis and Dermatomyositis, Polyneuritis Peripheral, Polyneuropathy Peripheral, Polyneuropathy and Polyradiculoneuropathy, Polyostotic Fibrous Dysplasia, Polyostotic Sclerosing Histiocytosis, Postmyelographic Arachnoiditis, Primary Progressive Multiple Sclerosis, Psoriasis, Radial Nerve Palsy, Radicular Neuropathy Sensory, Radicular Neuropathy Sensory Recessive, Reflex Sympathetic Dystrophy Syndrome, Relapsing-Remitting Multiple Sclerosis, Sensory Neuropathy Hereditary Type I, Sensory Neuropathy Hereditary Type II, Sensory Neuropathy Hereditary Type I, Sensory Radicular Neuropathy, Sensory Radicular Neuropathy Recessive, Sickle Cell Anemia, Sickle Cell Disease, Sickle Cell-Hemoglobin C Disease, Sickle Cell-Hemoglobin D Disease, Sickle Cell-Thalassemia Disease, Sickle Cell Trait, Spina Bifida, Spina Bifida Aperta, Spinal Arachnoiditis, Spinal Arteriovenous Malformation, Spinal Ossifying Arachnoiditis, Spinal Stenosis, Stenosis of the Lumbar Vertebral Canal, Still's Disease, Syringomyelia, Systemic Sclerosis, Talipes Calcaneus, Talipes Equinovarus, Talipes Equinus, Talipes Varus, Talipes Valgus, Tandem Spinal Stenosis, Temporal Arteritis/Giant Cell Arteritis, Temporal Arteritis, Tethered Spinal Cord Syndrome, Tethered Cord Malformation Sequence, Tethered Cord Syndrome, Tethered Cervical Spinal Cord Syndrome, Thalamic Pain Syndrome, Thalamic Hyperesthetic Anesthesia, Trigeminal Neuralgia, Variegate Porphyria, Vertebral Ankylosing Hyperostosis amongst others. These conditions may be treated with one or more local anesthetics as described herein.

For purposes of the present disclosure, in certain aspects the method of treatment induces an analgesic response to neuropathic and/or inflammatory pain being suffered by a mammalian, preferably human, patient. A patient, in this context, is also referred to as a "subject", "target" or "recipient". In this context the terms "analgesia" and "analgesic response" are intended to describe a state of reduced sensibility to pain at the site of application in certain aspects, and systemically in other aspects. To assess the level of reduction of sensibility to pain associated with the analgesia induced by the methods according to the present disclosure it is possible to conduct tests such as a VAS (visual analogue scales for pain intensity) questionnaire or the short form McGill pain questionnaire and/or verbal rating scales for pain intensity and/or measurement of tactile allodynia using von Frey hairs or similar device. These tests are standard tests within the art and would be well known to the skilled person.

### Local Anesthetic Embodiment

In certain preferred embodiments, the topical spray formulation of the invention includes a local anesthetic as an active agent. It is especially preferred in certain embodiments that the local anesthetic is bupivacaine hydrochloride. It has been unexpectedly found that the topical spray formulation is dose proportional only when the bupivacaine salt is used; the bupivacaine base incorporated into the topical spray compositions of the invention are not dose proportional.

In certain preferred embodiments, the bupivacaine hydrochloride topical spray formulation comprises a sufficient concentration of bupivacaine such that a dose from 0.1 mg to 50 mg is applied to the skin of a patient in a desired location. In certain preferred embodiments, the dose of bupivacaine is 10 mg, based on the bupivacaine hydrochloride salt. The dosage regimen can be, for example, 5 sprays (each spray containing 10 mg bupivacaine hydrochloride), in 5 distinct areas. In other preferred embodiments, from about 1 to about 30, or from about 1 to about 10, and preferably from about 3 to about 20 sprays of the topical spray formulation can be sprayed onto the skin about every 6 hours, twice a day, three times a day, once a day, or as needed.

Provides an in-vitro release of the bupivacaine of at least 1.0% after 2 hours, when tested via an in-vitro permeation study performed on a Strat-M synthetic membrane using In-line PermeGear ILC07 automatic diffusion cell system.

### DETAILED DESCRIPTION OF PREFERRED EMBODIMENTS

The following examples in accordance with the present invention are not to be construed as limiting the present invention in any manner and are only samples of the various formulations described herein.

### Example 1 (Comparative)

In Example 1, a bupivacaine HCl topical spray formulation was prepared using the ingredients set forth in Table 1.

Film forming sprays were prepared by adding target drug, non-volatile solvent and permeation enhancer to the solvent while stirring the solution to ensure complete dissolution of the drug and other excipients. The solvent used was ethanol (95 %). Having obtained a clear solution, polymer were added and other optional excipients. After addition of all excipients the solution was stirred to ensure complete dissolution and or hydration of polymer prior to use. The formulation was stored in glass vials sealed tightly with a cap or spray pump.

**Table 1**

| | **Example 1 mg/spray** |
|---|---|
| Bupivacaine HCl | 2.00 |
| Plastoid B | 6.00 |
| Eudragit EPO | 0.50 |
| Propylene Glycol | 5.00 |
| Transcutol P | 10.00 |
| Ethanol 95% | qs |
| Isopropyl Alcohol | qs |
| Menthol | 0.05 |
| | 100.00 |

The process for preparing the formulations is as follows:
Add drug, propylene glycol, Transcutol® (highly purified diethylene glycol monoethyl ether commercially available from Gattefosse; it is a highly purified hydrophilic solvent for drugs), Tween® 80 (polysorbate 80, a nonionic surfactant and emulsifier; Example 1), and menthol in ethanol with stirring and mix till clear solution formed. Add polymers (Plastoid B® and Eudragit® EPO) in isopropyl alcohol or water with stilling until uniform dispersion formed. Plastoid B® is a non-ionic copolymer of methyl methacrylate and butyl methacrylate, commercially available from Evonik. Eudragit® EPO is a cationic copolymer based on dimethylaminoethyl methacrylate, butyl methacrylate and methyl methacrylate, commercially available from Evonik. Add the drug clear solution in the polymeric dispersion under stirring till polymer dissolve or hydrate completely and form solution and or emulsion. Store the polymeric solution in glass bottle with cap or mechanical pump. The Eudragit EPO and Plastoid B were included as the polymers and ethanol and isopropyl alcohol were included as the solvent. 2mg aqueous solution of active substance was used as a positive control. The films were tested in in-vitro Franz Cell Strat-M synthetic membrane experiments. The results are set forth in Table 2 below:

**Table 2**

| **In-vitro cumulative drug permeation (ug/cm²)** | | |
|---|---|---|
| Time (Hr) | **Example 1** | **Control (2mg in water/spray)** |
| 0.5 | 0.98 | 1.33 |
| 1 | 6.17 | 34.35 |
| 2 | 10.73 | 101.92 |
| 4 | 15.72 | 167.49 |
| 6 | 20.83 | 197.59 |
| 24 | 32.77 | 219.40 |

It was observed that using the hydrophobic polymer such as Eudragit, about 1.2% of active permeates through the synthetic membrane over 24 hours. This is much lower than the amount of active that permeates from the aqueous control. The film that forms after spraying on the skin was peelable in nature and did not fully adhere to the skin or application site. Based on the film properties and rate of permeation it was decided not to use (solely) hydrophobic polymer in future studies.

### Examples 2-5

In Examples 2-5, bupivacaine HCl topical spray formulations were prepared using the ingredients set forth in Table 3, and further studies were performed using the hydrophilic polymers Avicel RC-591 (sodium CMC and MCC) and povidone K-30 (PVP) with water and or ethanol as solvent.

**Table 3**

| | **Example 2 mg/spray** | **Example 3 mg/spray** | **Example 4 mg/spray** | **Example 5 mg/spray** |
|---|---|---|---|---|
| Bupivacaine HCl | 2.00 | 8.00 | 2.00 | 2.00 |
| Avicel RC591 | 3.50 | 3.75 | 3.60 | |
| Povidone K30 | 3.50 | 3.75 | 3.60 | 6.00 |
| Propylene Glycol | 3.00 | 5.00 | 5.00 | 5.00 |
| Tween 80 | | | | 3.00 |
| Transcutol P | 5.00 | 5.00 | | 10.00 |
| Isopropyl Myristate | 5.00 | | 5.00 | 5.00 |
| Isostearic Acid | | 5.00 | | |
| Ethanol 95% | | | 40.75 | qs |
| Purified water | qs | qs | qs | 20.00 |
| Menthol | 0.05 | 0.05 | 0.05 | 0.05 |
| | 125.00 | 125.00 | 120.00 | 100.00 |

The process for preparing the formulations is as follows:
Add Avicel® RC591 (microcrystalline cellulose and carboxymethylcellulose sodium NF, Ph. Eur., commercially available from FMC Biopolymer) (except Example 5) and povidone K30 (polyvinylpyrollidone) in purified water with stirring and mix till polymer hydrates completely. Add drug, propylene glycol, transcutol (except Example 4), permeation enhancer and menthol in ethanol or purified water with stirring and mix till clear solution formed. Add the clear solution with drug in the polymeric dispersion while stirring till drug solution disperses uniformly with thixotropic polymeric gel. Store the polymeric thixotropic gel in glass bottle with cap or mechanical pump.

The films were tested in In-vitro Franz Cell Strat-M synthetic membrane experiments. The results are set forth in Table 4 below:

**Table 4**

| **In-vitro cumulative drug permeation (ug/cm²)** | | | | | | |
|---|---|---|---|---|---|---|
| **Time (Hr)** | **Lot 10** | **Lot 11** | **Example 3** | **Example 2** | **Control (2mg in water/spray)** | **Control (2mg in ethanol/spray)** |
| 0.5 | 0.33 | 0.30 | 3.22 | 2.68 | 1.33 | 13.94 |
| 1 | 0.50 | 0.31 | 5.15 | 10.97 | 34.35 | 97.09 |
| 2 | 0.75 | 0.49 | 9.18 | 76.94 | 101.92 | 483.12 |
| 4 | 0.89 | 0.64 | 18.37 | 371.43 | 167.49 | 1536.99 |
| 6 | 1.03 | 0.75 | 24.65 | 745.26 | 197.59 | 1828.75 |
| 24 | 1.65 | 1.04 | 100.65 | 1891.80 | 219.40 | 2008.52 |

From data obtained, it was concluded that if povidone is used as the polymer in the formulation containing a hydro-alcoholic solvent, active permeates at a much faster rate and extent than Avicel RC591. Avicel RC591 is composed of carboxymethylcellulose sodium which forms a gel like consistency and produces a larger droplet size upon spraying with a mechanical pump. It was concluded that using ethanol as solvent provides better permeation characteristics than water and a hydrophilic polymer is better than hydrophobic polymer with respect to permeation and adhesion to the skin.

### Examples 6-14

Based on the results set forth for Examples 1-5, it was decided that ethanol will be used as solvent and different permeation enhancers and hydrophilic polymers will be evaluated in the next series of studies. Eight different types of permeation enhancers (isopropyl myristate, oleth-2, oleic acid, 2-pyrolidone, isostearic acid, oleyly alcohol, polysorbate 80 and polyethylene glycol 600) were evaluated to understand the rate and extent of drug permeation. Bupivacaine topical spray formulations were prepared using the ingredients set forth in Table 5.

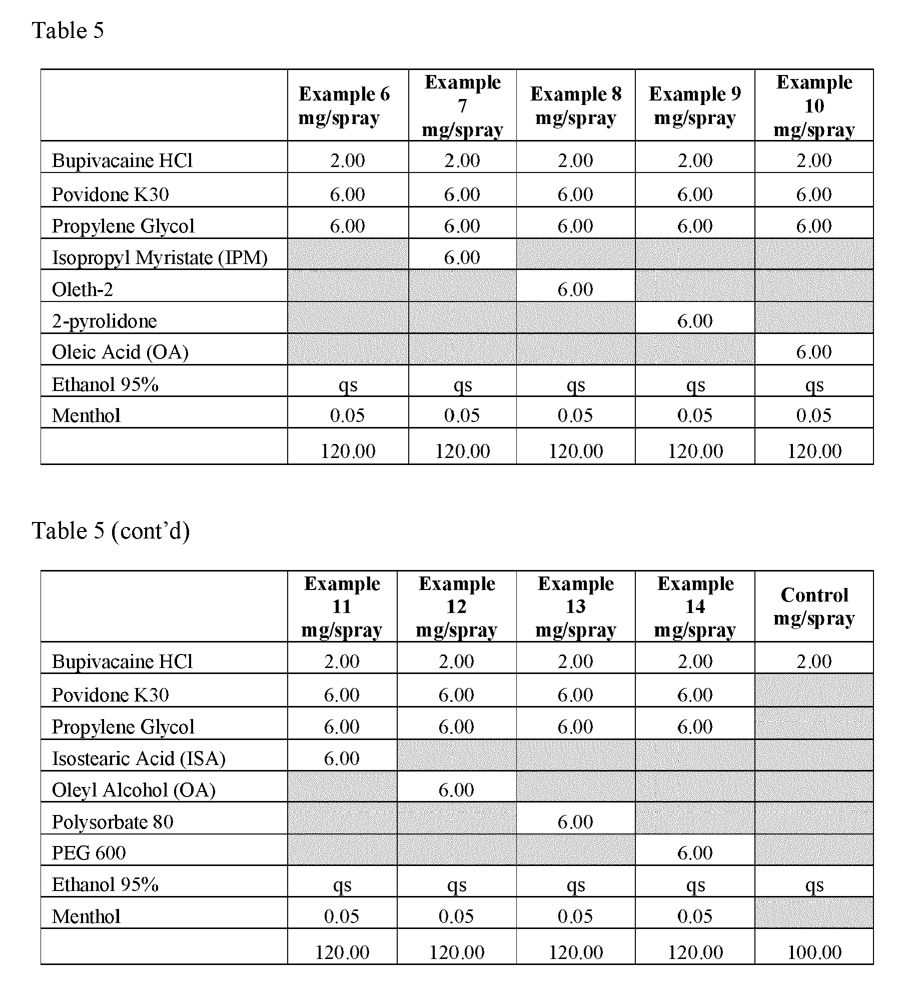

The process for preparing the formulations is as follows:
Add drug, propylene glycol, oleyl alcohol and menthol in ethanol under stirring and mix till clear solution formed. Add the film-forming polymer in the clear drug solution while stirring till polymer dissolves or hydrates completely. Store the polymeric film forming solution in glass bottle with cap or mechanical pump. Each of Examples 6-14 contain 5% w/w PVP in the formulation.

The films were tested in In-vitro Franz Cell Strat-M synthetic membrane experiments. The results are set forth in Table 6 below:

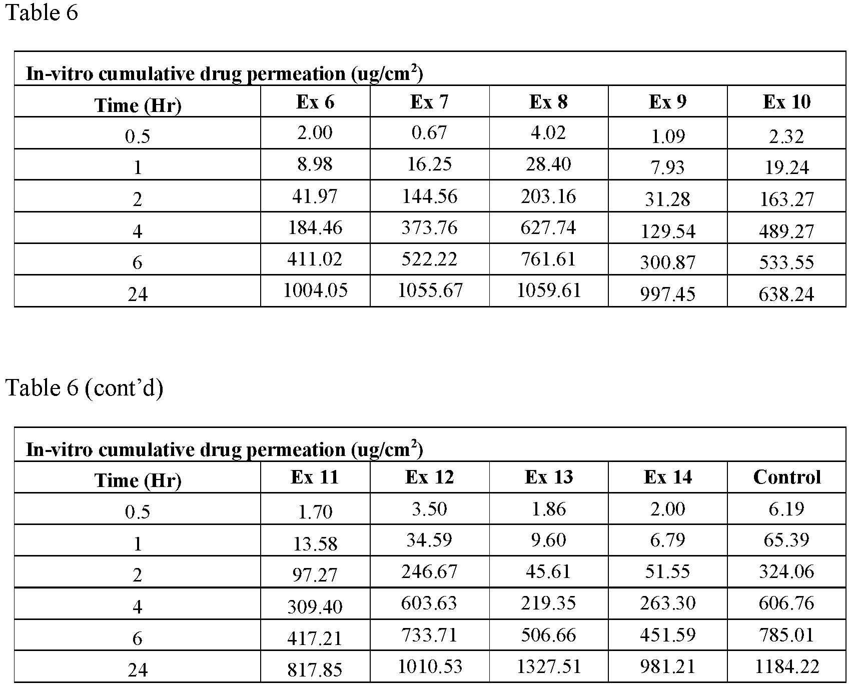

Based on data obtained, it can be concluded that permeation enhancer is very critical for initial permeation of active in the first two hours. Example 6 without any permeation enhancer permeates only 50ug/cm² of active in the first two hours and Example 12 permeates about 250ug/ cm² active in the first 2 hours. Also the type of permeation enhancer used is critical in achieving higher initial permeation of active.

### Examples 15 - 20

Based on the data obtained for Examples 6-14, oleyl alcohol and oleth-2 were further evaluated by varying the concentration in the formulation between 2.5 - 10% w/w. These two permeation enhancers were selected based on the initial rate and extent of active permeation through the membrane. In Examples 15 - 20, a bupivacaine topical spray formulation was prepared using the ingredients set forth in Table 7. Ethanol and povidone was used as solvent and polymer respectively.

**Table 7**

| | **Ex 15 mg/spray** | **Ex 16 mg/spray** | **Ex 17 mg/spray** | **Ex 18 mg/spray** | **Ex 19 mg/spray** | **Ex 20 mg/spray** |
|---|---|---|---|---|---|---|
| Bupivacaine HCl | 2.00 | 2.00 | 2.00 | 2.00 | 2.00 | 2.00 |
| Povidone K30 | 6.00 | 6.00 | 6.00 | 6.00 | 6.00 | 6.00 |
| Propylene Glycol | 6.00 | 6.00 | 6.00 | 6.00 | 6.00 | 6.00 |
| Oleth-2 | 3.00 | 9.00 | 12.00 | | | |
| Oleyl Alcohol | | | | 3.00 | 9.00 | 12.00 |
| Ethanol 95% | qs | qs | qs | qs | qs | qs |
| Menthol | 0.05 | 0.05 | 0.05 | 0.05 | 0.05 | 0.05 |
| | 120.00 | 120.00 | 120.00 | 120.00 | 120.00 | 120.00 |

The process for preparing the formulations is as follows:
Add drug, isopropyl myristate, polysorbate 80 and permeation enhancer in ethanol while stirring and mix till clear solution is formed. Add purified water drop by drop in drug:oil base solution with stirring until a nano-emulsion is formed. Add the film-forming polymer in the nano-emulsion while stirring till polymer dissolves or hydrates completely. Store the polymeric nano-emulsion in glass bottle with cap or mechanical pump.

The films were tested in In-vitro Franz Cell Strat-M synthetic membrane experiments. The results are set forth in Table 8 below:

**Table 8**

| **In-vitro cumulative drug permeation (ug/cm²)** | | | | | | |
|---|---|---|---|---|---|---|
| **Time (Hr)** | **Ex 15** | **Ex 16** | **Ex 17** | **Ex 18** | **Ex 19** | **Ex 20** |
| 0.5 | 2.13 | 3.70 | 4.97 | 1.76 | 3.08 | 2.76 |
| 1 | 12.55 | 33.42 | 41.34 | 11.74 | 39.21 | 26.32 |
| 2 | 95.08 | 220.63 | 234.56 | 182.63 | 302.15 | 216.83 |
| 4 | 459.51 | 635.85 | 599.48 | 648.62 | 649.14 | 633.11 |
| 6 | 679.53 | 769.52 | 725.15 | 735.48 | 706.03 | 704.43 |
| 24 | 1091.65 | 1152.72 | 1075.55 | 813.91 | 820.19 | 833.39 |

From the data, it was concluded that permeation enhancer concentration significantly improves the initial rate of permeation. In these Examples, the permeation enhancer concentration impacts Oleth-2 more than oleyl alcohol. Also, it was concluded that for Oleth-2, between 5 - 10 w/w concentration the impact of initial permeation rate was not that significant. It was decided that 7.5% w/w concentration of oleyl alcohol is an excellent permeation enhancer for the formulation.

### Examples 21 - 27

In Examples 21-27, a bupivacaine topical spray formulation was prepared using the ingredients set forth in Tables 9 and 10.

**Table 9**

| | **Example 21 mg/spray** | **Example 22 mg/spray** | **Example 23 mg/spray** |
|---|---|---|---|
| Bupivacaine | 8.00 | 8.00 | 8.00 |
| Povidone K30 | 6.00 | 6.00 | 6.00 |
| Propylene Glycol | 6.00 | 6.00 | 6.00 |
| Ethanol 95% | 90.94 | 90.94 | 90.94 |
| Menthol | 0.06 | 0.06 | 0.06 |
| Oleth - 2 | | 9.00 | |
| Oleic Acid | | | 9.00 |
| Isostearic Acid | 9.00 | | |
| Total | 120.00 | 120.00 | 120.00 |

**Table 10**

| | **Example 24 mg/spray** | **Example 25 mg/spray** | **Example 26 mg/spray** | **Example 27 mg/spray** |
|---|---|---|---|---|
| Bupivacaine Base | | 8.00 | 8.00 | 4.00 |
| Bupivacaine HCl | 8.00 | | | |
| Povidone K30 | 6.00 | 6.00 | | 6.00 |
| Copovidone | | | 6.00 | |
| Propylene Glycol | 6.00 | 6.00 | 6.00 | 6.00 |
| Ethanol 95% | 90.94 | 90.94 | 90.94 | 94.94 |
| Menthol | 0.06 | 0.06 | 0.06 | 0.06 |
| Oleyl Alcohol | 9.00 | 9.00 | | 9.00 |
| Isopropyl myristate | | | 9.00 | |
| | 120.00 | 120.00 | 120.00 | 120.00 |

The process for preparing the formulations is as follows:
Add drug, propylene glycol, permeation enhancer and menthol in ethanol while stirring and mix till a clear solution is formed. Add the film-forming polymer in the clear drug solution while stirring till polymer dissolves or hydrates completely. Store the polymeric in glass bottle with cap or mechanical pump.

In-vitro permeation studies were performed on the formulations of Examples 21-27 on Mattek Epiderm and or cadaver skin using an In-line PermeGear ILC07 automatic diffusion cell system. While designing in-vitro skin permeation studies using human cadaver skin, the donor age group was considered in order to represent the target patient population. For development studies, human cadaver skin from a white male donor between age group of 50 - 60 years was selected. Human cadaver skin was received from the New York Firefighter Skin Bank as a cryopreserved split thickness skin allograft. A skin graft contains the epidermis and partial dermis that simulates the in-vivo diffusion barrier layer. The human cadaver skin was stored as received at temperature of -30°C. Before performing the in-vitro skin permeation studies the skin was submerged in the phosphate buffer solution at room temperature for 15 minutes. The in-vitro skin permeation studies were performed using a PermeGear ILC07 automatic diffusion cell system. Cumulative (µg/cm²) drug permeated through synthetic membrane with respect to time is provided in Table 11 below.

**Table 11**

| **Hr** | **Cumulative Drug Permeation ug/cm²** | | | | | | |
|---|---|---|---|---|---|---|---|
| | **E-21** | **E-22** | **E-23** | **E-24** | **E-25** | **E-26** | **E-27** |
| 0 | 0 | 0 | 0 | 0 | 0 | 0 | 0 |
| 0.5 | 5 | 5 | 5 | 5 | 8 | 16 | 7 |
| 1 | 9 | 11 | 12 | 12 | 25 | 36 | 16 |
| 2 | 28 | 50 | 56 | 32 | 91 | 103 | 64 |
| 4 | 175 | 257 | 262 | 179 | 266 | 278 | 281 |
| 6 | 313 | 391 | 402 | 337 | 424 | 433 | 420 |
| 24 | 1020 | 955 | 1063 | 971 | 1792 | 1399 | 786 |

The cumulative (µg/cm²) drug permeated through human cadaver skin with respect to time is provided in Table 12 below with respect to Examples 24 and 25. Three different human cadaver skin donors were used to understand the impact of skin type on permeation rate. Seven diffusion cells were used per formulation per donor and the average of 21 permeation data points per formulation is reported in Table 12 below.

**Table 12**

| **Time (Hr)** | **Cumulative Drug Permeation ug/cm²** | | | | | | | |
|---|---|---|---|---|---|---|---|---|
| | **Example 24** | | | | **Example 25** | | | |
| | **Donor 1** | **Donor 2** | **Donor 3** | **Average** | **Donor 1** | **Donor 2** | **Donor 3** | **Average** |
| 0 | 0 | 0 | 0 | 0 | 0 | 0 | 0 | 0 |
| 0.5 | 10 | 16 | 25 | 17 | 22 | 14 | 4 | 13 |
| 1 | 18 | 52 | 43 | 38 | 38 | 34 | 8 | 27 |
| 2 | 36 | 177 | 86 | 100 | 86 | 90 | 19 | 65 |
| 4 | 223 | 651 | 289 | 387 | 244 | 234 | 59 | 179 |
| 6 | 489 | 963 | 472 | 641 | 396 | 373 | 110 | 293 |
| 24 | 1787 | 2080 | 1056 | 1641 | 1356 | 1633 | 639 | 1209 |

Based on the above data, it was concluded that bupivacaine hydrochloride (Example 24) demonstrates superior permeation than bupivacaine base (Example 25) in the three different skin donors.

### Example 28

In Example 28, particle size distribution data for the bupivacaine metered dose transdermal film spray of Example 24 is provided in Table 13 below:

**Table 13: Bupivacaine topical spray droplet size distribution data**

| Unit ID | Dv(10), µm | Dv(50), µm | Dv(90), µm | %Droplets < 10 µm | Pump Delivery (mg) |
|---|---|---|---|---|---|
| Example 24 - first spray attempt | 27.63 | 56.77 | 121.9 | 1.497 | 126.1 |
| Example 24 - second spray attempt | 26.01 | 54.04 | 114.9 | 1.593 | 125.8 |
| Example 24 - third spray attempt | 24 | 52.01 | 112.4 | 1.740 | 125.0 |
| **Mean** | **25.88** | **54.27** | **116.4** | **1.610** | **125.6** |
| **SD** | **1.82** | **2.39** | **4.9** | **0.12** | **0.6** |
| **% CV** | **7.0** | **4.4** | **4.2** | **7.6** | **0.5** |

### Example 29: In-Vivo Study

An in-vivo study was performed in healthy rats to evaluate drug release from a bupivacaine topical bio-adhesive film forming spray made in accordance with Example 24. A total of 4 sprays were applied (two sprays on the back and two sprays on the abdominal area of the animal) using a metered dose mechanical spray pump. Blood samples were collected at 15, 30 minutes, 1, 2, 4, 8 and 24 hours. All samples were analyzed using a validated analytical LC-MS method.

The permeation or release of the drug following topical application from the invention is characterized by a biphasic release profile, the first phase comprising an immediate release of drug from the drug-solvent micro-droplet that is facilitated by the small (approximately 50 microns) spray droplet size and a subsequent second controlled release phase comprising release of drug from the bio-adhesive film formed, upon evaporation of the solvent system.

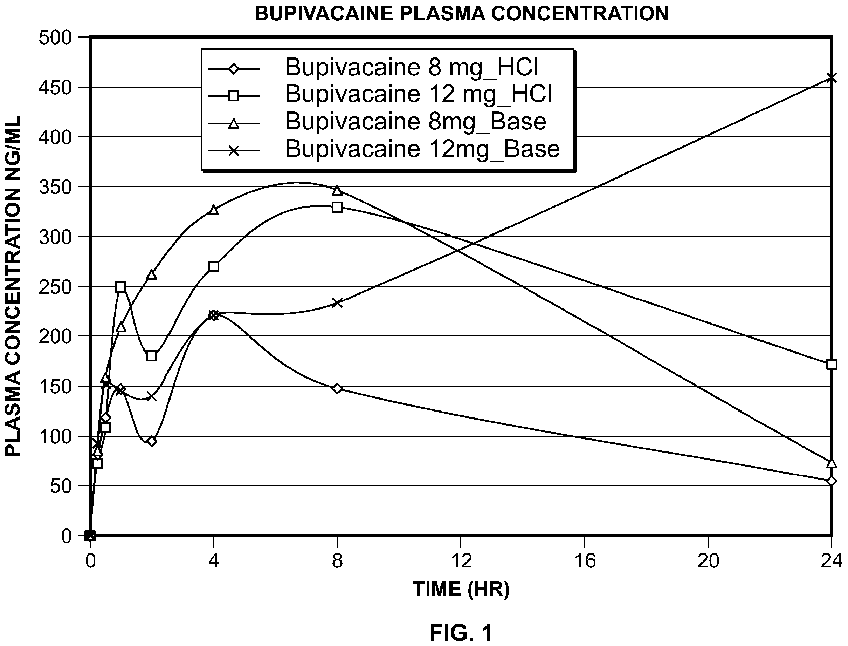

The results obtained are reported in Table 14 and Figs 1 - 4. It can be concluded from the results that the topical/transdermal drug delivery formulations of the invention have a bi-phasic release profile and the drug release is dose proportional. Both the 8 and 12 mg strengths show the first phase of drug release at about 1 hour after spray application, signifying that spray pattern and droplet size is very critical in achieving immediate drug permeation and fast onset of action. The second slower phase of drug release observed between 4 - 8 hours after spray application indicates that drug gradually permeates from the polymeric bio-adhesive film in a controlled manner. This observed in-vivo bi-phasic drug release through the topical/ transdermal route is unique to this invention - a drug containing polymeric bio-adhesive film spray. It is also clear from the data that the lag in Cmax during the second phase of drug release at higher drug concentrations signifies a prolongation in effect as dosage strength is increased.

**Table 14: Plasma concentration of drug at various time-points**

| **Time (Hr)** | **Average drug plasma concentration ng/ml** | | |
|---|---|---|---|
| | **Bupivacaine HCl 8 mg** | **Bupivacaine HCl 12 mg** | **Placebo** |
| 0 | 1 | 0 | 0 |
| 0.25 | 82 | 73 | 23 |
| 0.5 | 119 | 109 | 35 |
| 1 | 148 | 249 | 50 |
| 2 | 95 | 180 | 48 |
| 4 | 221 | 270 | 39 |
| 8 | 147 | 329 | 49 |
| 24 | 55 | 172 | 8 |

Figure 1 depicts an in-vivo drug plasma concentration vs time plot of individual rats treated with a bupivacaine metered dose transdermal film spray in accordance with the present invention, bupivacaine HCl 8 mg (n=5), bupivacaine HCl 12 mg (n=3), bupivacaine base 8 mg (n=7), and bupivacaine base 12 mg (n=3).

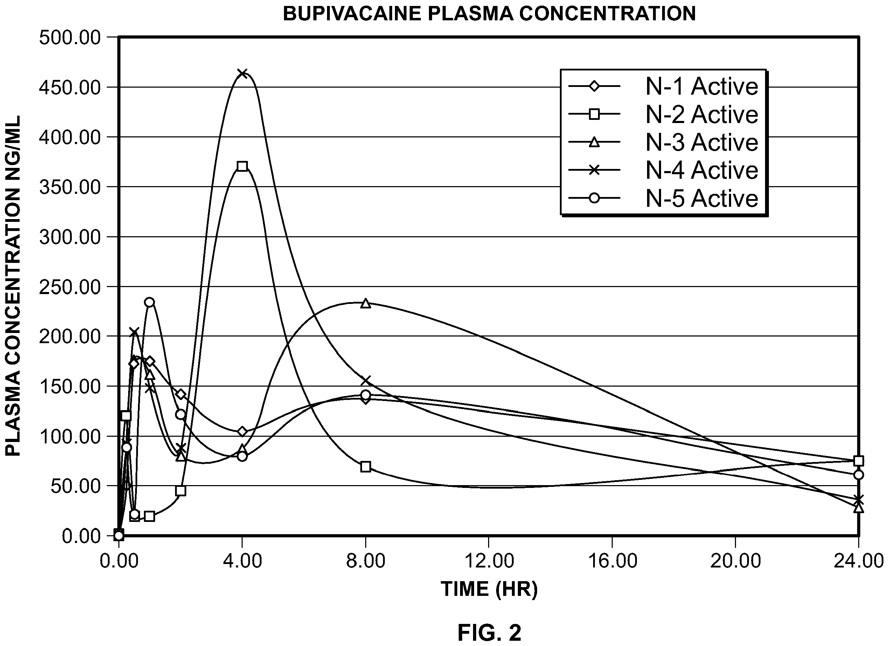

Figure 2 depicts an in-vivo drug plasma concentration vs time plot of individual rats treated with a bupivacaine metered dose transdermal film spray in accordance with the present invention.

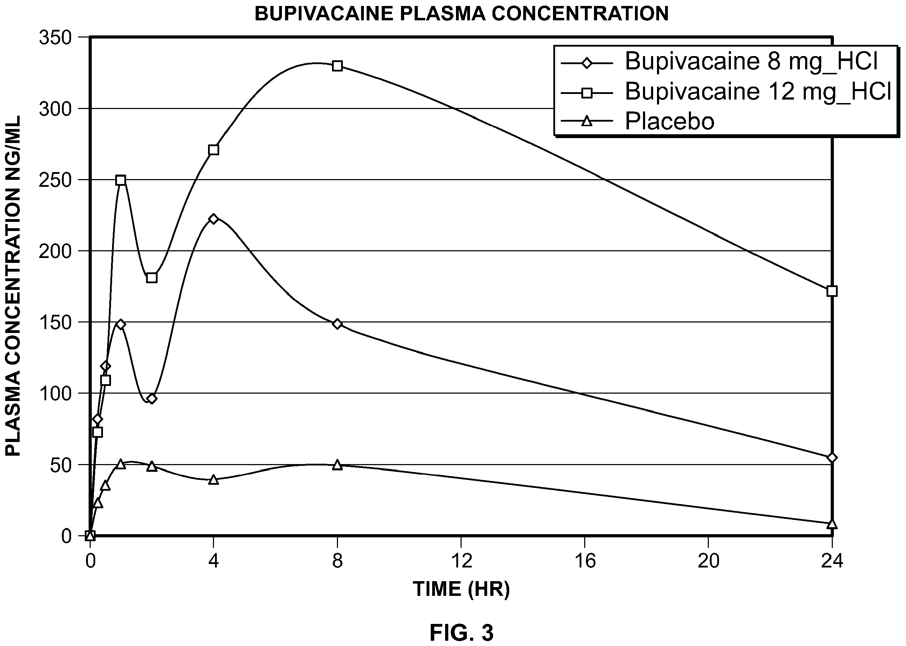

Figure 3 depicts an in-vivo average drug plasma concentration vs time plot of 8 mg, 12 mg and placebo of individual rats treated with a bupivacaine metered dose transdermal film spray in accordance with the present invention.

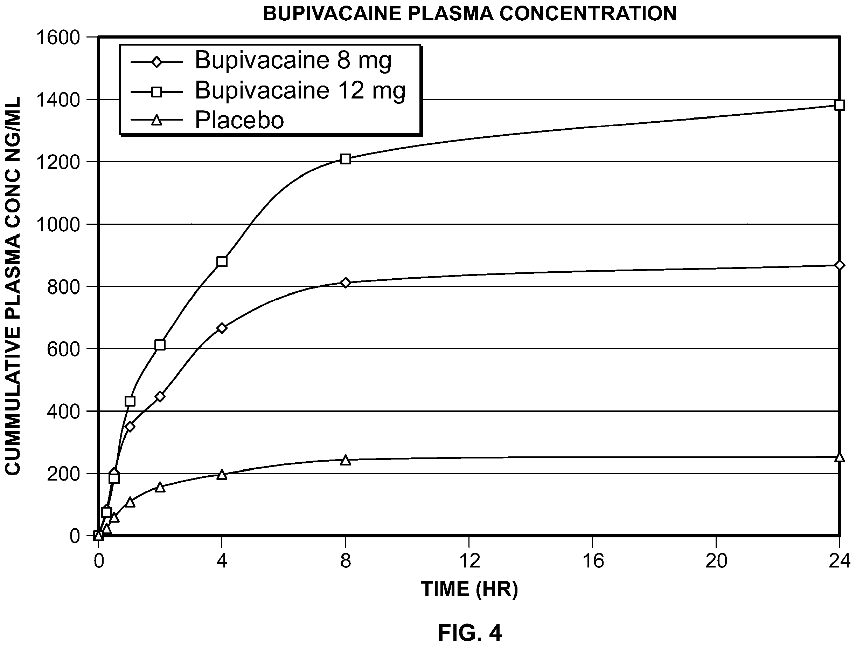

Figure 4 depicts an in-vivo average cumulative drug plasma concentration vs time plot of 8 mg, 12 mg and placebo of individual rats treated with a bupivacaine metered dose transdermal film spray in accordance with the present invention.

### Examples 30-32

In Examples 30-32, local anesthetic topical spray formulations were prepared using the ingredients set forth in Table 15 for bupivacaine base, ropivacaine base and lidocaine HCl.

**Table 15**

| | **Example 30 mg/spray** | **Example 31 mg/spray** | **Example 32 mg/spray** |
|---|---|---|---|
| Bupivacaine Base | 2.00 | | |
| Ropivacaine Base | | 2.00 | |
| Lidocaine HCl | | | 2,00 |
| Povidone K30 | 6.00 | 6.00 | 6.00 |
| Propylene Glycol | 6.00 | 6.00 | 6.00 |
| Oleyl Alcohol | 6.00 | 6.00 | 6.00 |
| Ethanol 95% | qs | qs | qs |
| Menthol | 0.05 | 0.05 | 0.05 |
| | 120.00 | 120.00 | 120.00 |

The process for preparing the formulations is as follows:
Add drug, propylene glycol, permeation enhancer and menthol in ethanol while stirring and mix till a clear solution is formed. Add the film-forming polymer in the clear drug solution while stirring till polymer dissolves or hydrates completely. Store the polymeric in glass bottle with cap or mechanical pump.

The films were tested in In-vitro Franz Cell Strat-M synthetic membrane experiments. The results are set forth in Table 16 below:

**Table 16**

| **In-vitro cumulative drug permeation (ug/cm²)** | | | |
|---|---|---|---|
| **Time (Hr)** | **Ex 30 - (Bupi Base)** | **Ex 31- (Ropi Base)** | **Ex 32 - (Lido HCl)** |
| 0.5 | 1.78 | 0.56 | 2.25 |
| 1 | 8.04 | 10.81 | 41.75 |
| 2 | 41.97 | 76.95 | 283.76 |
| 4 | 172.65 | 314.58 | 519.55 |
| 6 | 272.59 | 463.83 | 569.71 |
| 24 | 566.76 | 744.90 | 637.97 |

### Example 33

In Examples 33a-c, a testosterone topical spray formulation was prepared using the ingredients set forth in Table 17.

**Table 17**

| | **Example 33a mg/spray** | **Example 33b mg/spray** | **Example 33c mg/spray** |
|---|---|---|---|
| Testosterone | 10.00 | 30.00 | 30.00 |
| Povidone K30 | 6.00 | 12.00 | 12.00 |
| Propylene Glycol | 6.00 | 12.00 | 12.00 |
| Ethanol 95% | qs | qs | qs |
| Menthol | 0.06 | 0.06 | 0.12 |
| Oleyl Alcohol | 9.00 | 12.00 | |
| Isopropyl Myristate | | | 6.00 |
| Isostearic Acid | | | 6.00 |

The method of preparation is similar to the procedure set forth above for Examples 2-5.

### Example 34

In Examples 34a-c, a ketamine topical spray formulation was prepared using the ingredients set forth in Table 18.

**Table 18**

| | **Example 34a mg/spray** | **Example 34b mg/spray** | **Example 34C mg/spray** |
|---|---|---|---|
| Ketamine HCl | 15.00 | 30.00 | 30.00 |
| Bupivacaine HCl | 6.00 | | 6.00 |
| Povidone K30 | 6.00 | 12.00 | 12.00 |
| Propylene Glycol | 6.00 | 12.00 | 12.00 |
| Ethanol 95% | qs | qs | qs |
| Menthol | 0.06 | 0.06 | 0.06 |
| Oleyl Alcohol | 9.00 | 12.00 | 12.00 |

The method of preparation is similar to the procedure set forth above for Examples 2-5.

### Example 35

In Examples 35a-b, an estradiol topical spray formulation was prepared using the ingredients set forth in Table 19.

**Table 19**

| | **Example 35a mg/spray** | **Example 35b mg/spray** |
|---|---|---|
| Estradiol | 0.5 | 2.0 |
| Povidone K30 | 6.00 | 6.00 |
| Propylene Glycol | 6.00 | 6.00 |
| Ethanol 95% | qs | qs |
| Menthol | 0.06 | 0.06 |
| Oleyl Alcohol | 9.00 | |
| Isopropyl Myristate | | 9.00 |

The method of preparation is similar to the procedure set forth above for Examples 2-5.

### Example 36

In Example 36, a fentanyl topical spray formulation was prepared using the ingredients set forth in Table 20.

**Table 20**

| | **mg/spray** |
|---|---|
| Fentanyl | 0.1-2.0 |
| Povidone K30 | 6.00 |
| Propylene Glycol | 6.00 |
| Ethanol 95% | Qs |
| Menthol | 0.06 |
| Oleyl Alcohol | 9.00 |

The method of preparation is similar to the procedure set forth above for Examples 2-5.

### Example 37

In Examples 37a-b, a methylphenidate topical spray formulation was prepared using the ingredients set forth in Table 21.

**Table 21**

| | **Example 37a mg/spray** | **Example 37b mg/spray** |
|---|---|---|
| Methylphenidate | 2.0 | 20.0 |
| Povidone K30 | 6.00 | 6.00 |
| Propylene Glycol | 6.00 | 6.00 |
| Ethanol 95% | Qs | qs |
| Isopropyl Alcohol | | 20.00 |
| Menthol | 0.06 | 0.06 |
| Oleyl Alcohol | 9.00 | |
| Isopropyl Myristate | | 12.00 |

The method of preparation is similar to the procedure set forth above for Examples 2-5.

### Example 38

In Examples 38a-b, a neomycin topical spray formulation was prepared using the ingredients set forth in Table 22.

**Table 22**

| | **Example 38a mg/spray** | **Example 38b mg/spray** |
|---|---|---|
| Neomycin | 10.00 | 20.0 |
| Povidone K30 | 6.00 | 6.00 |
| Propylene Glycol | 6.00 | 6.00 |
| Ethanol 95% | qs | qs |
| Purified water | | 40.00 |
| Menthol | 0.06 | 0.06 |
| Oleyl Alcohol | 9.00 | |

The method of preparation is similar to the procedure set forth above for Examples 2-5.

### Example 39

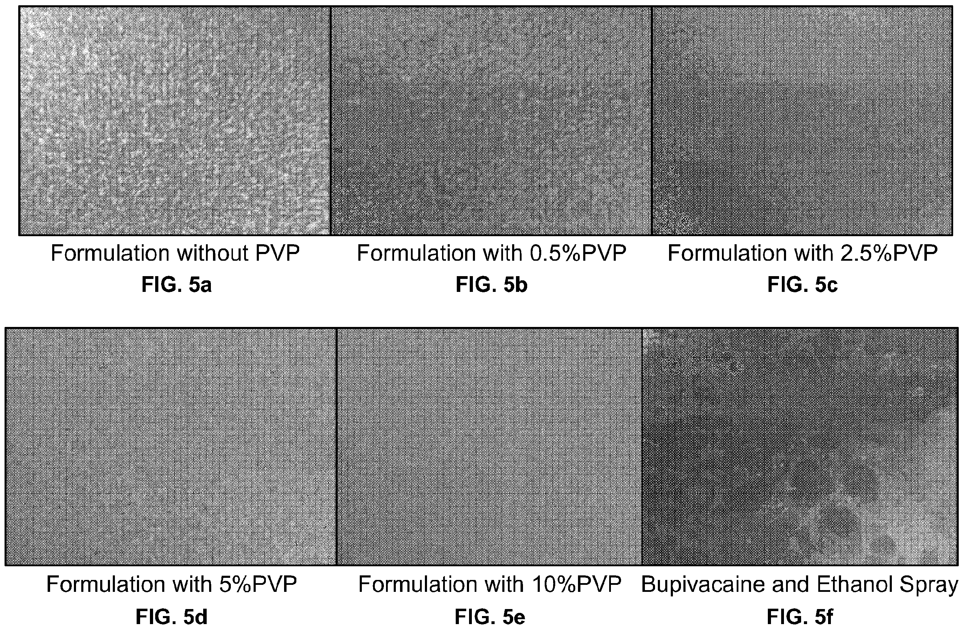

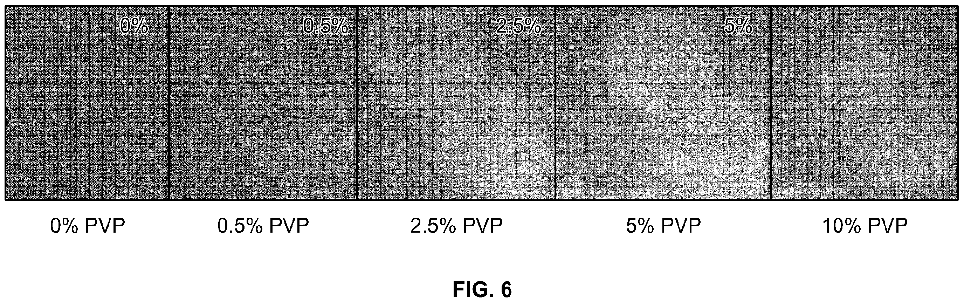

Bupivacaine topical spray formulations were prepared using different concentrations of polyvinyl pyrrolidone (PVP) (0.0 %, 0.5 %, 2.5%, 5% and 10%) and sprayed over glass slides to evaluate the rate and extent of bupivacaine crystal precipitation after topical spray film formation. Those containing 0.0 % and 0.5 % PVP are not part of the invention. Figures 5 and 6 visually illustrate the bupivacaine topical spray film characteristics. More specifically, Figure 5 depicts bupivacaine topical spray film characteristics in formulations a) without PVP (significant bupivacaine crystals observed); b) formulation with 0.5% PVP (significant bupivacaine crystals observed; c) formulation with 2.5% PVP; d) formulation with 5% PVP; e) formulation with 10% PVP; and f) bupivacaine and ethanol spray (significant bupivacaine crystals observed). It was observed that the rate and extent of bupivacaine crystal precipitation significantly decrease with increase in the formulation polymer concentration. It was also observed that the uniformity and integrity of film increases with the increase in polymer concentration. More specifically, Figure 6 depicts the spray pattern and film uniformity of bupivacaine spray formulations according to the present invention containing different concentrations of the precipitation inhibiting polymer PVP (0% PVP, 0.5% PVP; 2.5% PVP; 5% PVP; and 10% PVP. It was concluded that a PVP concentration in the formulation of between 2.5% and 10% significantly inhibits bupivacaine precipitation and forms a uniform film after solvent evaporation.

### Example 40

In Examples 40a-b, a bupivacaine HC1 topical spray formulation was prepared using the ingredients set forth in Table 23.

**Table 23**

| | **Example 40a mg/spray** | **Example 40b mg/spray** |
|---|---|---|
| Bupivacaine HC1 | 10.56 | 10.56 |
| Povidone K30 | 6.00 | 6.00 |
| Propylene Glycol | 6.00 | 6.00 |
| Ethanol 95% | 88.14 | 88.38 |
| Menthol | 0.06 | 0.06 |
| Oleyl Alcohol | 9.00 | 9.00 |
| Ascorbic Acid | 0.24 | |
| | 120.00 | 120.00 |

The process for preparing the formulations is as follows:
Add drug, propylene glycol, permeation enhancer, ascorbic acid (antioxidant agent) and menthol in ethanol while stirring and mix till a clear solution is formed. Add the film-forming polymer in the clear drug solution while stirring till polymer dissolves or hydrates completely. Store the polymeric in glass bottle with cap or mechanical pump.

### Example 41

In Example 41, a single dose crossover study to evaluate the pharmacokinetics and relative bioavailability of 3 doses of bupivacaine topical spray and bupivacaine injectable in healthy male and female volunteers was conducted. The pharmacokinetic study was performed in 12 healthy human volunteers to assess the safety and tolerability of bupivacaine topical spray delivery system and estimate the duration time of local effect perceived by the healthy volunteers following single escalating doses of bupivacaine topical spray. The reference therapy was a single 30 mg dose (a total of 6 ml, 3 injections of 2ml) of bupivacaine HCl administered subcutaneously.

The test formulation was a bupivacaine hydrochloride 10 mg topical spray (corresponding to 8.88 mg of bupivacaine base) prepared in accordance with Example 40b. The reference formulation was Sensorcaine® (bupivacaine hydrochloride) 0.5% 10 mL.

A single subcutaneous dose of Treatment-A or a single topical dose of Treatment-B, -C, or -D was administered according to the following randomization scheme: Treatment-A: Single dose of 30 mg: 3 subcutaneous injections (2 mL each) of Sensorcaine® (Bupivacaine Hydrochloride) 0.5% 10 mL (Reference); Treatment-B: Single dose of 30 mg: 3 sprays of GTX101 (Bupivacaine Hydrochloride 10 mg Topical Spray; Test); Treatment-C: Single dose of 50 mg: 5 sprays of GTX101 (Bupivacaine Hydrochloride 10 mg Topical Spray; Test); Treatment-D: Single dose of 70 mg: 7 sprays of GTX101 (Bupivacaine Hydrochloride 10 mg Topical Spray; Test).

Two treatments (Treatment-A and either Treatment-B, -C or -D) were to be administered to 12 subjects as described in Table 24. Subjects arrived at the clinical site at least 10 hours before dosing. After a supervised overnight fast, subjects received a standard breakfast before drug administration following which, a single subcutaneous dose of bupivacaine hydrochloride 30 mg or a single topical dose of bupivacaine hydrochloride 30 mg, 50 mg or 70 mg was administered in the morning. Subjects were allowed to leave the clinical site after the 24-hour post dose blood draw. The wash-out period was at least 3 calendar days.

**Table 24: Study Sequences**

| | **Period 1** | **Period 2** |
|---|---|---|
| Sequence 1 (n= 2) | Treatment-A | Treatment-B |
| Sequence 2 (n= 2) | Treatment-B | Treatment-A |
| Sequence 3 (n= 2) | Treatment-A | Treatment-C |
| Sequence 4 (n= 2) | Treatment-C | Treatment-A |
| Sequence 5 (n= 2) | Treatment-A | Treatment-D |
| Sequence 6 (n= 2) | Treatment-D | Treatment-A |

All 12 volunteers completed the study. In each study period, 21 blood samples were collected. The first sample was collected prior to drug administration while the others were collected up to 24 hours after the drug administration. Additionally, skin irritation was assessed at the application site approximately 5 minutes, 6 hours, and 24 hours after dosing for each treatment. At each time point the local skin reaction was assessed and recorded on a specific form included in the Case Report Form. All 12 subjects showed no changes in their skin irritation assessments, all dermal responses were scored as 0; there was no evidence of irritation and no other effects were observed.

### Perception Analysis (Efficacy Analysis)

Subjects were questioned if they felt the sensation of a Q-Tip (Yes / No) at every 30 minutes after drug application for the first 8 hours or until sensation returned whichever came first. Two Q-Tip tests were performed one at the site of application and another at a location away from the site of application. The Q-Tip test was performed by lightly touching the Q-Tip on the surface of skin. All local effects perceived by the subjects that persisted 8 hours after drug administration were reported as adverse events. Both the reference and test products shows similar results for the loss in sensation after Q-tip analysis which suggest that the novel bupivacaine topical spray is equally efficacious as reference subcutaneous injectable product. The Q-tip perception analysis results are reported in Table 25.

**Table 25: Summary of Q-Tip perception analysis**

| **Subject** | **Reported loss of Q-tip sensation (Yes / No) in the first 8 hours after dosing** | |
|---|---|---|
| | **Topical Spray** | **SC Injection** |
| 1 | Yes | No |
| 2 | No | Yes |
| 3 | No | Yes |
| 4 | Yes | Yes |
| 5 | Yes | No |
| 6 | Yes | No |
| 7 | Yes | Yes |
| 8 | No | No |
| 9 | No | Yes |
| 10 | Yes | Yes |
| 11 | Yes | No |
| 12 | No | Yes |
| Total # of subjects reported loss in Q-tip sensation | 7 out of 12 | 7 out of 12 |
| % Subject reported loss in Q-tip sensation | 58.3% | 58.3% |

The pharmacokinetic results are reported in Table 26. Tmax is shown in units of hours and Cmax is in units of ng/mL.

**Table 26: Summary of plasma bupivacaine pharmacokinetic parameters**

| **Parameter (Units)** | **Treatment A (n=12) 30 mg SC** | | **Treatment B (n=4) 30 mg Spray** | | **Treatment C (n=4) 50 mg Spray** | | **Treatment D (n=4) 70 mg Spray** | |
|---|---|---|---|---|---|---|---|---|
| | **Mean** | **(C.V. %)** | **Mean** | **(C.V. %)** | **Mean** | **(C.V. %)** | **Mean** | **(C.V. %)** |
| Cₘₐₓ (ng/mL) | 129.31 | (41.2) | 1.03 | NC | 2.41 | (44.6) | 1.55 | (31.3) |
| Tₘₐₓ (hours) | 0.33 | (0.17-0.67) | 24.00 | NC | 24.00 | (24.00-24.05) | 24.00 | (2.00-24.00) |

| | | | | | | | | |
|---|---|---|---|---|---|---|---|---|
| NC Not calculated | | | | | | | | |

From a single dose application over 24 hours, a mean maximum plasma concentration of 2.41 ng/mL for topical spray formulation and 129.31 ng/mL for reference injectable product was observed. From the above clinical study, it can be concluded that the novel bupivacaine topical spray formulation is effective, safe and well tolerated.

### Example 42

In Example 42, an open-label study to evaluate the single dose pharmacokinetics of bupivacaine topical spray in healthy male and female volunteers was conducted. The pharmacokinetic study was performed in 10 healthy human volunteers to assess the safety, tolerability of a 100 mg dose of topical bupivacaine administered as 10 topical sprays. The study was a single center, non-randomized, single dose, open-label, one period, one treatment design. A 100 mg dose of bupivacaine was administered topically (10 sprays applied in a similar distribution covering the skin surface at the T10 dermatome). The bupivacaine spray provided about 10 mg bupivacaine per spray. The formulation was prepared in accordance with Example 40b.

All 10 volunteers completed the study. 29 blood samples were collected per subject. The first sample was collected prior to drug administration while the others were collected up to 504 hours after drug administration. Additionally, skin irritation was assessed at the application site approximately 5 minutes, 6 hours, and 24 hours after dosing for each treatment. At each time point the local skin reaction was assessed and recorded on a specific form included in the Case Report Form. All 10 subjects showed no changes in their skin irritation assessments, all dermal responses were scored as 0; there was no evidence of irritation and no other effects were observed.

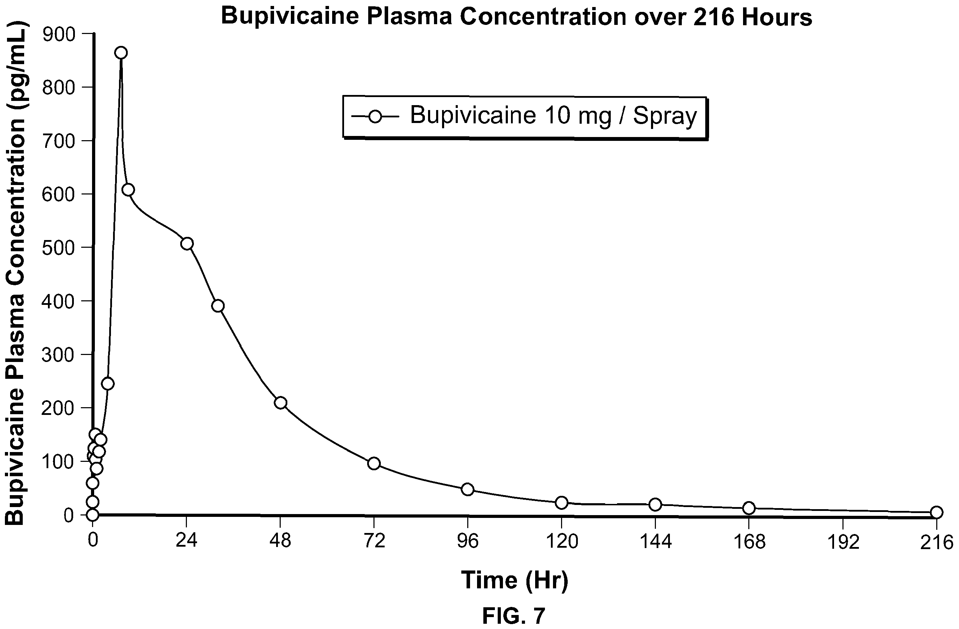

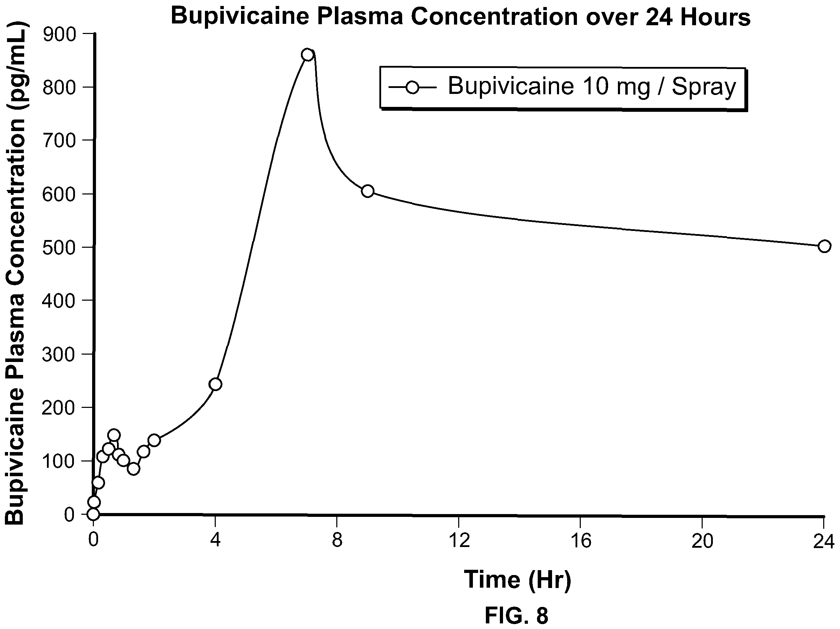

The pharmacokinetic results are reported in Figures 7 and 8 and Table 26. Tmax is shown in units of hours and Cmax is in units of pg/mL. Figure 7 is a graph depicting the bupivacaine plasma concentration (pg/mL) over 216 hours. Figure 8 is a graph depicting the bupivacaine plasma concentration over 24 hours.

**Table 27: Summary of plasma bupivacaine pharmacokinetic parameters**

| **Parameter (Units)** | **Mean** | **(C.V. %)** |
|---|---|---|
| Cₘₐₓ (pg/mL) | 1248.72 | 92.1 |
| Tₘₐₓ (hours) | 11.97 | (0.67-24.00) |
| AUC_{0-T} (pg·h/mL) | 29493.41 | 66.5 |
| T_{half} (hours) | 33.86 | 75.6 |

From a single dose application over 504 hours, a mean maximum plasma concentration of 1248 pg/mL was observed. It can be concluded from Figures 7 and 8 that this topical/transdermal drug delivery technology invention has a bi-phasic release profile similar to that observed in the rat study. The first phase of drug releases at about 30 - 40 minutes after spray application, signifying that spray pattern and droplet size are critical to achieving immediate drug permeation and fast onset of action. The second slower phase of drug release observed between 7 - 24 hours after spray application indicates that drug gradually permeates from the polymeric bio-adhesive film in a controlled manner. This observed in-vivo bi-phasic drug release through the topical/ transdermal route is unique to the present invention - a drug-containing polymeric bio-adhesive topical film spray. It is also clear from the data that the lag in Cmax during the second phase of drug release at higher drug concentrations signifies a prolongation in effect as dosage strength is increased. Table 28 provides blood sample times and plasma concentration breakdowns for both peaks in the individual subjects.

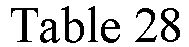

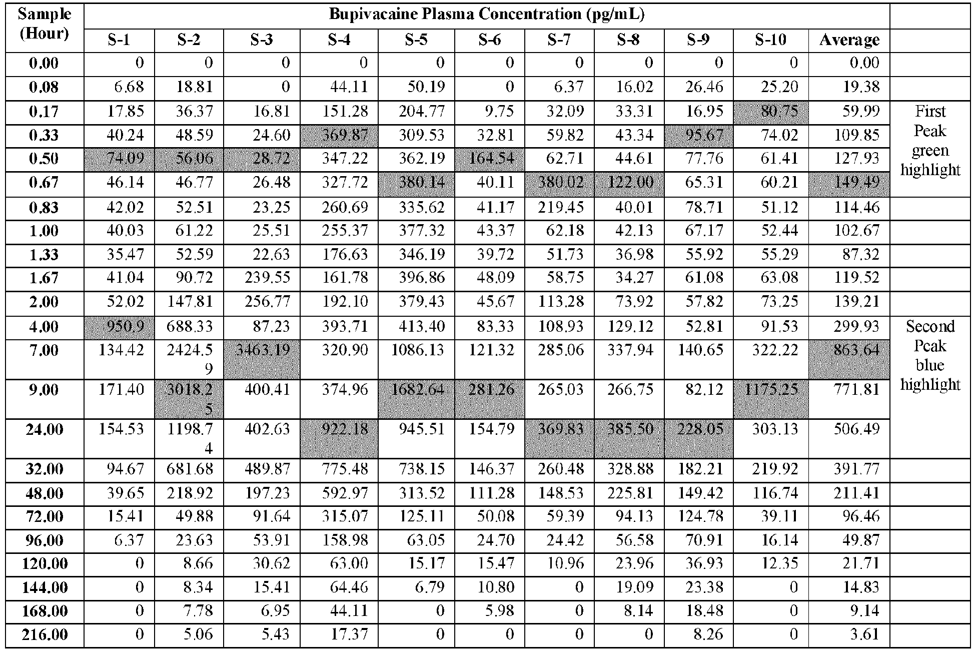

### Examples 43a-b

In-vitro permeation studies were conducted on formulations prepared in accordance with the invention.

In Example 43a, the formulation of Example 12 was tested for cumulative drug permeation per cm² from a Strat-M synthetic membrane. Over the first two hours, the drug permeation ranged from 10 µg/ cm² to 500 µg/ cm². Over 24 hours, the drug permeation ranged from 10 µg/ cm² to 6500 µg/ cm².

In Example 43b, the formulation of Example 24 was tested for cumulative drug permeation per cm² from Mattek and human cadaver skin membrane. Over the first two hours, the drug permeation ranged from 10 µg/ cm² to 500 µg/ cm². Over 24 hours, the drug permeation ranged from 10 µg/ cm² to 6500 µg/ cm².

### Examples 44a-b

In-vivo permeation studies were conducted on formulations prepared in accordance with the invention.

In Example 44a, the formulation of Example 24 was tested for cumulative drug permeation per cm² on rat skin. Over the first two hours, the drug permeation ranged from 10 ng/cm² to 500 ng/cm². Over 24 hours, the drug permeation ranged from 10 ng/cm² to 6500 ng/cm².

In Example 44b, the formulation of Example 40b was tested for cumulative drug permeation per cm² on human skin. Over the first two hours, the drug permeation ranged from 10 ng/cm² to 500 ng/cm². Over 24 hours, the drug permeation ranged from 10 ng/cm² to 6500 ng/cm².

### Example 45

Solubility studies to find out concentration at which the drug is present at saturation levels were performed as follows: The drug (bupivacaine hydrochloride 2, 4, 8, 10, 12 & 16 mg) was added to, propylene glycol, oleyl alcohol and menthol in ethanol under stirring and mixing till clear solution is formed. The film-forming polymer in the clear drug solution was added while stirring till polymer dissolves or hydrates completely. In the case of 16 mg of drug solution the drug precipitated out from clear solution after standing. Other solutions were clear and no precipitate was observed. It can be concluded that drug saturation was achieved at a concentration of about 12 mg per spray.

### Conclusion

While the invention has been described in connection with certain embodiments, it is not intended to limit the invention to the particular forms set forth, but on the contrary, it is intended to cover such alternatives and modifications as may be included within the scope of the invention as defined by the following claims.

## Claims

1. A polymeric film forming topical spray formulation, comprising a hydrophilic film forming polymer, an active agent, a drug crystal precipitation inhibiting agent, wherein the hydrophilic film forming polymer and the drug crystal precipitation inhibiting agent are the same and it is povidone in an amount from 2.5 to 20% by weight, the amount of povidone being effective to prevent or substantially prevent the active agent included in the formulation from precipitating, a pharmaceutically acceptable permeation enhancer for the active agent, and a volatile solvent in a concentration from 20 to 99% of the formulation, by weight, the formulation when sprayed on and set on a site on human skin providing a breathable, bioadhesive and microporous film in which the active agent is supersaturated and further providing a biphasic release of the active agent(s) such that the formulation provides a first peak concentration of the active agent at 0.25 to 1.5 hours after application of the topical spray on the skin of a human subject, and provides a second peak concentration of the active agent at 4 hours to 12 hours after application of the topical spray on the skin of a human subject.

2. The topical spray formulation of claim 1, wherein the active agent(s) is selected from the group consisting of bupivacaine base, bupivacaine hydrochloride, and a combination thereof.

3. The topical spray formulation of claim 1, wherein the active agent is selected from the group consisting of an anesthetic, steroid, an opioid, a non-steroidal anti-inflammatory agent (NSAID), a central nervous system stimulant, an anti-bacterial, and combinations of any of the foregoing.

4. The topical spray formulation of claim 1, wherein a unit dose comprises a plurality of spray droplets, wherein 10% of the spray droplets in the unit dose have a mean diameter of 26 µm ± 20 µm, 50% of the spray droplets in the unit dose have a mean diameter of 55 µm ± 20 µm, and 90% of the spray droplets in the unit dose have a mean diameter of 116 µm ± 40 µm.

5. The topical spray formulation of claim 1, which provides an in-vitro cumulative drug permeation from 10 ug/cm² to 500 ug/cm² after 2 hours, and a cumulative drug permeation from 10 µg/cm² to 6500 µg/cm² after 24 hours, or an in-vivo cumulative drug permeation on human skin from 10 ng/cm² to 500 ng/cm² in-vitro after 2 hours, and a cumulative drug permeation from 10 ng/cm² to 6500 ng/cm² after 24 hours.

6. A unit dose of a topical spray pharmaceutical formulation comprising a polymeric solution, emulsion or suspension of povidone in an amount from 2.5 to 20% by weight, a therapeutically effective amount of a supersaturated active agent, and a pharmaceutically acceptable permeation enhancer dispersed in a pharmaceutically acceptable hydroalcoholic solvent, the unit dose comprising a plurality of spray droplets, wherein 10% of the spray droplets in the unit dose have a mean diameter of 26 µm ± 20 µm, 50% of the spray droplets in the unit dose have a mean diameter of 55 µm ± 20 µm, and 90% of the spray droplets in the unit dose have a mean diameter of 116 µm ± 40 µm, the unit dose topical spray provides a film surface area from 1 cm² to 40 cm² per spray and sets as a microporous, breathable and bioadhesive film when the hydroalcoholic solvent evaporates and provides a biphasic release of the supersaturated active agent.

7. The unit dose of claim 6, wherein the active agent comprises from 0.5 to 40 mg bupivacaine hydrochloride.

8. The unit dose of claim 6, wherein the biphasic release provides a first peak concentration of the active agent at from 0.17 to 0.67 hours and the second peak concentration of the active agent at from 4 to 24 hours after application of the unit dose on human skin.

9. The unit dose of claim 7, wherein the bupivacaine concentration in the formulation is supersaturated and the supersaturated drug concentration lasts for a time period from 1 to 24 hours in vivo to achieve increased bioavailability.

10. The unit dose of claim 6, wherein 10% of the spray droplets in the unit dose have a mean diameter of 26 µm ± 1.82 µm, 50% of the spray droplets in the unit dose have a mean diameter of 55 µm ± 2.39 µm, and 90% of the spray droplets in the unit dose have a mean diameter of 116 µm ± 4.9 µm.

11. The unit dose of claim 7, wherein the first peak concentration occurs at from 0.17 to 0.67 hours after the unit dose is sprayed onto the human subject, and the second peak concentration occurs at from 4 to 24 hours after the unit dose is sprayed onto the human subject.

12. The unit dose of claim 6, wherein the unit dose provides a first peak plasma concentration from 29 pg/ml to 380 pg/ml bupivacaine, and a second peak plasma concentration from 864 pg/ml to 3463 pg/ml bupivacaine.

## Patentansprüche

1. Polymerische filmbildende topische Sprayformulierung, die ein Polymer, das einen hydrophilen Film bildet, einen Wirkstoff, ein Arzneimittelkristallausfällungsinhibierendes Mittel umfasst, wobei das hydrophile filmbildende Polymer und das Arzneimittelkristallausfällungsinhibierende Mittel dasselbe sind und es sich um Pivodon in einer Menge von 2,5 bis 20 Gew.-% handelt, wobei die Pivodon-Menge wirksam ist, um den Wirkstoff, der in der Formulierung eingeschlossen ist, am Ausfällen zu hindern oder im Wesentlichen zu hindern, einen pharmazeutisch akzeptablen Permeationsverstärker für den Wirkstoff, und ein flüchtiges Lösemittel in einer Konzentration von 20 bis 99 Gew.-% der Formulierung, wobei, wenn die Formulierung auf einer Stelle auf menschlicher Haut aufgesprüht wird und sich absetzt, ein atemfähiger, bioadhäsiver und mikroporöser Film bereitgestellt wird, in dem der Wirkstoff übersättigt ist, und weiter eine zweiphasige Freigabe des Wirkstoffs (der Wirkstoffe) bereitgestellt wird, sodass die Formulierung eine erste Spitzenkonzentration des Wirkstoffs 0,25 bis 1,5 Stunden nach dem Auftragen des topischen Sprays auf der Haut eines menschlichen Subjekts bereitstellt, und eine zweite Spitzenkonzentration des Wirkstoffs 4 Stunden bis 12 Stunden nach dem Auftragen des topischen Sprays auf der Haut des menschlichen Subjekts bereitstellt.

2. Topische Sprayformulierung nach Anspruch 1, wobei der Wirkstoff (die Wirkstoffe) aus der Gruppe ausgewählt ist (sind), die aus Bupivacain-Basis, Bupivacain-Hydrochlorid und einer Kombination davon besteht.

3. Topische Sprayformulierung nach Anspruch 1, wobei der Wirkstoff aus der Gruppe ausgewählt ist, die aus einem Anästhetikum, Steroid, einem Opioid, einem nichtsteroidalen entzündungshemmenden Mittel (NSAID), einem das zentrale Nervensystem stimulierenden Mittel, einem antibakteriellen Mittel und Kombinationen der Vorstehenden besteht.

4. Topische Sprayformulierung nach Anspruch 1, wobei eine Einzeldosis eine Vielzahl von Spraytröpfchen umfasst, wobei 10 % der Spraytröpfchen in der Einzeldosis einen mittleren Durchmesser von 26 µm ± 20 µm aufweisen, 50 % der Spraytröpfchen in der Einzeldosis einen mittleren Durchmesser von 55 µm ± 20 µm aufweisen, und 90 % der Spraytröpfchen in der Einzeldosis einen mittleren Durchmesser von 116 µm ± 40 µm aufweisen.

5. Topische Sprayformulierung nach Anspruch 1, die eine kumulative In-Vitro-Arzneimittelpermeation von 10 ug/cm² bis 500 ug/cm² nach 2 Stunden, und eine kumulative Arzneimittelpermeation von 10 µg/cm² bis 6500 µg/cm² nach 24 Stunden oder eine kumulative In-Vivo-Arzneimittelpermeation auf menschlicher Haut von 10 ng/cm² bis 500 ng/cm² in vitro nach 2 Stunden, und eine kumulative Arzneimittelpermeation von 10 ng/cm² bis 6500 ng/cm² nach 24 Stunden bereitstellt.

6. Einzeldosis einer topischen pharmazeutischen Sprayformulierung, die eine polymerische Lösung, Emulsion oder Suspension von Povidon in einer Menge von 2,5 bis 20 Gew.-%, eine therapeutisch wirksame Menge eines übersättigten Wirkstoffs und einen pharmazeutisch akzeptablen Permeationsverstärker, der in einem pharmazeutisch akzeptablen hydroalkoholischen Lösemittel verteilt ist, umfasst, wobei die Einzeldosis eine Vielzahl von Spraytröpfchen umfasst, wobei 10 % der Spraytröpfchen in der Einzeldosis einen mittleren Durchmesser von 26 µm ± 20 µm aufweisen, 50% der Spraytröpfchen in der Einzeldosis einen mittleren Durchmesser von 55 µm ± 20 µm aufweisen, und 90 % der Spraytröpfchen in der Einzeldosis einen mittleren Durchmesser von 116 µm ± 40 µm aufweisen, wobei der topische Einzeldosis-Spray eine Filmoberfläche von 1cm² bis 40 cm² pro Spray bereitstellt, und sich als ein mikroporöser, atemfähiger und bioadhäsiver Film absetzt, wenn das hydroalkoholische Lösemittel verdunstet und eine zweiphasige Freigabe des übersättigten Wirkstoffs bereitstellt.

7. Einzeldosis nach Anspruch 6, wobei der Wirkstoff 0,5 bis 40 mg Bupivacain-Hydrochlorid umfasst.

8. Einzeldosis nach Anspruch 6, wobei die zweiphasige Freisetzung eine erste Spitzenkonzentration des Wirkstoffs 0,17 bis 0,67 Stunden und die zweite Spitzenkonzentration des Wirkstoffs 4 bis 24 Stunden nach dem Auftragen der Einzeldosis auf menschlicher Haut bereitstellt.

9. Einzeldosis nach Anspruch 7, wobei die Bupivacain-Konzentration in der Formulierung übersättigt ist, und die übersättigte Arzneimittelkonzentration während eines Zeitraums von 1 bis 24 Stunden in vivo anhält, um gesteigerte Bioverfügbarkeit zu erreichen.

10. Einzeldosis nach Anspruch 6, wobei 10 % der Spraytröpfchen in der Einzeldosis einen mittleren Durchmesser von 26 µm ± 1,82 µm aufweisen, 50 % der Spraytröpfchen in der Einzeldosis einen mittleren Durchmesser von 55 µm ± 2,39 µm aufweisen, und 90 % der Spraytröpfchen in der Einzeldosis einen mittleren Durchmesser von 116 µm ± 4,9 µm aufweisen.

11. Einzeldosis nach Anspruch 7, wobei die erste Spitzenkonzentration 0,17 bis 0,67 Stunden nach dem Sprühen der Einzeldosis auf das menschliche Subjekt auftritt, und die zweite Spitzenkonzentration 4 bis 24 Stunden nach dem Sprühen der Einzeldosis auf das menschliche Subjekt auftritt.

12. Einzeldosis nach Anspruch 6, wobei die Einzeldosis eine erste Spitzenplasmakonzentration von 29 pg/ml bis 380 pg/ml Bupivacain, und eine zweite Spitzenplasmakonzentration von 864 pg/ml bis 3463 pg/ml Bupivacain bereitstellt.

## Revendications

1. Formulation de pulvérisation topique filmogène polymère, comprenant un polymère filmogène hydrophile, un agent actif, un agent inhibiteur de précipitation de cristaux médicamenteux, dans laquelle le polymère filmogène hydrophile et l'agent inhibiteur de précipitation de cristaux médicamenteux sont identiques et il s'agit de povidone en une quantité allant de 2,5 à 20 % en poids, la quantité de povidone étant efficace pour empêcher ou empêcher sensiblement l'agent actif inclus dans la formulation de précipiter, un amplificateur de perméation acceptable sur le plan pharmaceutique pour l'agent actif, et un solvant volatil en une concentration allant de 20 à 99 % de la formulation, en poids, la formulation lorsqu'elle est pulvérisée sur et figée sur un site sur la peau humaine fournissant un film respirant, bioadhésif et microporeux dans lequel l'agent actif est sursaturé et fournissant en outre une libération biphasique du ou des agent(s) actif(s) de sorte que la formulation fournit un premier pic de concentration de l'agent actif de 0,25 à 1,5 heure après application de la pulvérisation topique sur la peau d'un sujet humain, et fournit un deuxième pic de concentration de l'agent actif de 4 heures à 12 heures après application de la pulvérisation topique sur la peau d'un sujet humain.

2. Formulation de pulvérisation topique selon la revendication 1, dans laquelle le(s) agent(s) actif(s) est(sont) sélectionné(s) à partir du groupe consistant en une base de bupivacaïne, du chlorhydrate de bupivacaïne, et une combinaison de ceux-ci.

3. Formulation de pulvérisation topique selon la revendication 1, dans laquelle l'agent actif est sélectionné à partir du groupe consistant en un anesthésique, un stéroïde, un opioïde, un agent anti-inflammatoire non stéroïdien (AINS), un stimulant du système nerveux central, un antibactérien, et des combinaisons de quelconques des précédents.

4. Formulation de pulvérisation topique selon la revendication 1, dans laquelle une dose unitaire comprend une pluralité de gouttelettes de pulvérisation, dans laquelle 10 % des gouttelettes de pulvérisation dans la dose unitaire présentent un diamètre moyen de 26 µm ± 20 µm, 50 % des gouttelettes de pulvérisation dans la dose unitaire présentent un diamètre moyen de 55 µm ± 20 µm, et 90 % des gouttelettes de pulvérisation dans la dose unitaire présentent un diamètre moyen de 116 µm ± 40 µm.

5. Formulation de pulvérisation topique selon la revendication 1, qui fournit une perméation médicamenteuse cumulée in vitro allant de 10 ng/cm² à 500 ng/cm² après 2 heures, et une perméation médicamenteuse cumulée allant de 10 ng/cm² à 6 500 ng/cm² après 24 heures, ou une perméation médicamenteuse cumulée in vivo sur la peau humaine allant de 10 ng/cm² à 500 ng/cm² in vitro après 2 heures, et une perméation médicamenteuse cumulée allant de 10 ng/cm² à 6500 ng/cm² après 24 heures.

6. Dose unitaire d'une formulation pharmaceutique de pulvérisation topique comprenant une solution, émulsion ou suspension polymère de povidone en une quantité allant de 2,5 à 20 % en poids, une quantité efficace sur le plan thérapeutique d'un agent actif sursaturé, et un amplificateur de perméation acceptable sur le plan pharmaceutique dispersé dans un solvant hydroalcoolique acceptable sur le plan pharmaceutique, la dose unitaire comprenant une pluralité de gouttelettes de pulvérisation, dans laquelle 10 % des gouttelettes de pulvérisation dans la dose unitaire présentent un diamètre moyen de 26 µm ± 20 µm, 50 % des gouttelettes de pulvérisation dans la dose unitaire présentent un diamètre moyen de 55 µm ± 20 µm, et 90 % des gouttelettes de pulvérisation dans la dose unitaire présentent un diamètre moyen de 116 µm ± 40 µm, la pulvérisation topique en dose unitaire fournit une superficie de film allant de 1 cm² à 40 cm² par pulvérisation et se fige en tant que film microporeux, respirant et bioadhésif lorsque le solvant hydroalcoolique s'évapore et fournit une libération biphasique de l'agent actif sursaturé.

7. Dose unitaire selon la revendication 6, dans laquelle l'agent actif comprend de 0,5 à 40 mg de chlorhydrate de bupivacaïne.

8. Dose unitaire selon la revendication 6, dans laquelle la libération biphasique fournit un premier pic de concentration de l'agent actif après 0,17 à 0,67 heure et le deuxième pic de concentration de l'agent actif de 4 à 24 heures après application de la dose unitaire sur la peau humaine.

9. Dose unitaire selon la revendication 7, dans laquelle la concentration en bupivacaïne dans la formulation est sursaturée et la concentration en médicament sursaturée dure pendant un laps de temps allant de 1 à 24 heures in vivo pour obtenir une biodisponibilité accrue.

10. Dose unitaire selon la revendication 6, dans laquelle 10 % des gouttelettes de pulvérisation dans la dose unitaire présentent un diamètre moyen de 26 µm ± 1,82 µm, 50 % des gouttelettes de pulvérisation dans la dose unitaire présentent un diamètre moyen de 55 µm ± 2,39 µm, et 90 % des gouttelettes de pulvérisation dans la dose unitaire présentent un diamètre moyen de 116 µm ± 4,9 µm.

11. Dose unitaire selon la revendication 7, dans laquelle le premier pic de concentration se produit de 0,17 à 0,67 heure après que la dose unitaire est pulvérisée sur le sujet humain, et le deuxième pic de concentration se produit de 4 à 24 heures après que la dose unitaire est pulvérisée sur le sujet humain.

12. Dose unitaire selon la revendication 6, dans laquelle la dose unitaire fournit un premier pic de concentration plasmatique allant de 29 pg/ml à 380 pg/ml de bupivacaïne, et un deuxième pic de concentration plasmatique allant de 864 pg/ml à 3 463 pg/ml de bupivacaïne.
